(19) **Europäisches Patentamt / European Patent Office / Office européen des brevets**

(11) **EP 3 940 061 B1**

(12) **EUROPEAN PATENT SPECIFICATION**

(45) Date of publication and mention of the grant of the patent:
**15.07.2026 Bulletin 2026/29**

(21) Application number: **20773511.9**

(22) Date of filing: **13.03.2020**

(51) International Patent Classification (IPC):
**C12N 5/0775** (2010.01)   **C12N 5/078** (2010.01)
**C12N 5/0783** (2010.01)   **C12N 1/04** (2006.01)

(52) Cooperative Patent Classification (CPC):
**C12N 5/0644; C12N 1/04; C12N 5/0636;
C12N 5/0663;** C12N 2500/38; C12N 2501/60;
C12N 2501/606; C12N 2506/45

(86) International application number:
**PCT/JP2020/011002**

(87) International publication number:
**WO 2020/189538 (24.09.2020 Gazette 2020/39)**

(54) **STORAGE LIQUID FOR MAMMALIAN CELLS**

KONSERVIERUNGSFLÜSSIGKEIT FÜR SÄUGETIERZELLEN

LIQUIDE DE STOCKAGE POUR CELLULES DE MAMMIFÈRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.03.2019 JP 2019047852**

(43) Date of publication of application:
**19.01.2022 Bulletin 2022/03**

(73) Proprietors:
• **Megakaryon Corporation
Kyoto-shi, Kyoto 600-8813 (JP)**
• **Otsuka Pharmaceutical Factory, Inc.
Naruto-shi, Tokushima 772-8601 (JP)**

(72) Inventors:
• **TOMIZUKA, Junko
Kyoto-shi, Kyoto 600-8813 (JP)**
• **SHIGEMORI, Tomohiro
Kyoto-shi, Kyoto 600-8813 (JP)**
• **WATANABE, Natsuki
Naruto-shi, Tokushima 772-8601 (JP)**
• **TAKENAWA, Taichi
Naruto-shi, Tokushima 772-8601 (JP)**
• **SHIRAKAWA, Chikage
Naruto-shi, Tokushima 772-8601 (JP)**
• **NISHIMURA, Masuhiro
Naruto-shi, Tokushima 772-8601 (JP)**

• **FUJITA, Yasutaka
Naruto-shi, Tokushima 772-8601 (JP)**
• **SAWAMOTO, Osamu
Naruto-shi, Tokushima 772-8601 (JP)**

(74) Representative: **Mathys & Squire
32 London Bridge Street
The Shard
London SE1 9SG (GB)**

(56) References cited:
WO-A1-2014/208053    WO-A1-2017/065280
WO-A1-2018/097226    WO-A2-2012/012682
JP-A- 2016 538 859    JP-A- 2018 515 099
JP-A- 2018 516 955    JP-A- H 057 619
US-A- 5 104 787    US-A1- 2016 205 923
US-B2- 8 927 202

• KUCHLER ET AL: "Milieu for Maintaining and Growing Animal Cells In Vitro", 1 January 1977, BIOCHEMICAL METHODS IN CELL CULTURE AND VIROLOGY,, PAGE(S) 45, XP001345237
• LEE E J ET AL: "The effect of human serum albumin on the extended storage of human oral keratinocyte viability under mild hypothermia", CRYOBIOLOGY, ACADEMIC PRESS INC, US, vol. 50, no. 1, 1 February 2005 (2005-02-01), pages 103 - 111, XP004745170, ISSN: 0011-2240, DOI: 10.1016/J.CRYOBIOL.2004.12.001

**EP 3 940 061 B1**

- MAIESE K ET AL: "Nicotinamide: necessary nutrient emerges as a novel cytoprotectant for the brain", TRENDS IN PHARMACOLOGICAL SCIENCES, ELSEVIER, HAYWARTH, GB, vol. 24, no. 5, 1 May 2003 (2003-05-01), pages 228 - 232, XP004427195, ISSN: 0165-6147
- HIGA, YUKIE : "No. 16 Effect of preserved PC on platelet function by addition of water-soluble vitamins, About the effect of vitamin C", JOURNAL OF THE JAPAN SOCIETY OF BLOOD TRANSFUSION, vol. 48, no. 1, 2002, pages 48, XP009530637, ISSN: 0546-1448
- GIAMMONA, L. M. ET AL.: "Nicotinamide (vitamin B3) increases the polyploidisation and proplatelet formation of cultured primary human megakaryocytes", BRITISH JOURNAL OF HAEMATOLOGY, vol. 135, 2006, pages 554 - 566, XP055135582, DOI: 10.1111/j.1365-2141.2006.06341.x
- XU, Y. ET AL.: "Tannic acid attenuated irradiation-induced apoptosis in megakaryocytes", EXPERIMENTAL CELL RESEARCH, vol. 370, 2018, pages 409 - 416, XP085447194, DOI: 10.1016/j.yexcr.2018.07.003

## Description

### Technical Field

The present invention relates to the use of a preservation solution for preserving mammalian cells, comprising niacin or a salt of niacin (hereinafter, also referred to as "niacin, etc.")and ascorbic acid or a salt of ascorbic acid (hereinafter, also referred to as "ascorbic acid, etc.").

### Background Art

Platelet formulations are administered at the time of bleeding ascribable to surgery or wound as well as to patients having thrombocytopenia. Although the platelet formulations are currently manufactured from blood obtained by blood donation, a shortage of the platelet formulations is concerned due to a reduced amount of blood donated in association with change in the composition of populations.

In the case where blood donors have infections by bacteria or the like, the blood might be contaminated with the bacteria. Therefore, there is a risk of infection by the administration of platelet formulations contaminated with the bacteria. Hence, a method for manufacturing platelets *in vitro* has been developed (non-patent document 1), and various techniques have been established for stable large-scale production (patent documents 1 and 2).

The platelet formulations are manufactured by mixing platelets with a proper preservation solution and packing the mixture into a blood bag or the like. Since the form and functions of platelets vary rapidly depending on low temperatures, the platelet formulations are preserved under shaking at room temperature (20 to 24°C) for use. However, it is known that various problems such as decrease in the functions of platelets arise during preservation. Thus, their preservation over a long period of time has been impossible. Hence, there is a demand for the development of a preservation solution that prevents decrease in the functions of platelets and is capable of extending the preservation periods of platelet formulations.

Mesenchymal stem cells are somatic stem cells present in bone marrow, fat tissue, etc. and have the ability to differentiate into bone, cartilage and fat, etc. Hence, the mesenchymal stem cells have received attention as a promising cell source for transplantation treatment, and the development of culture and preservation methods is underway (patent documents 3 and 4). Recently, various cancer immunotherapies using T cells have been developed and are under clinical trial (non-patent document 2). However, it has been reported that when T cells are freeze-preserved, the expression of PD-1 (immune checkpoint molecule) on T cell membranes is markedly decreased (non-patent document 3). Thus, there is a demand for the development of a method for non-frozen preservation of T cells.

### Prior Art Documents

### Patent Documents

Patent Document 1: WO2017/077964
Patent Document 2: PCT/JP2018/034667
Patent Document 3: WO2017/073656
Patent Document 4: Japanese unexamined Patent Application Publication No. 2018-153196

Non-patent Document 1: Takayama et. al., 2008, Blood, 111: 5298-5306

Non-patent Document 2: Tyler et. al., 2013, Blood, 121: 308-317

Non-patent Document 3: Campbell et. al., 2009, Clin vaccine immunol, 16: 1648-53

### Summary of the Invention

### Object to be Solved by the Invention

An object of the present invention is to provide a novel preservation solution that is superior in preservation stability to a conventional preservation solution and can preserve mammalian cells over a long period of time.

**Means to Solve the Object**

The present inventors have conducted diligent studies to attain the aforementioned object and consequently completed the present invention by finding that (1) when platelets are preserved under shaking in an isotonic solution supplemented with ascorbic acid (hereinafter, also referred to as "vitamin C" or "VC"), the deterioration of the platelets is suppressed as compared with a conventional platelet preservation solution, (2) when niacin (hereinafter, also referred to as "vitamin B3" or "VB3") is added in combination with VC to the isotonic solution, the deterioration of the platelets is more efficiently suppressed, (3) the isotonic solution supplemented with VC and VB3 is also effective for the non-frozen preservation of other mammalian cells such as mesenchymal stem cells, megakaryocytes, and T cells, and (4) such a cell preservation effect brought about by VC and VB3 is inhibited by riboflavin (vitamin B2; hereinafter, also referred to as "VB2").

Specifically, the present invention relates to: (1) use of a preservation solution for preserving a platelet or a megakaryocyte for 1 to 15 days at 0 to 40°C, wherein the preservation solution comprises 10 to 5000 mg/L niacin or a salt of niacin, and 10 to 8000 mg/L of ascorbic acid or a salt of ascorbic acid, and does not comprise vitamin B2 or a salt of vitamin B2; (2) use according to the aforementioned (1), wherein a concentration of niacin or the salt of niacin is 30 to 3000 mg/L; (3) use according to the aforementioned (1), wherein a concentration of niacin or the salt of niacin is 120 to 1200 mg/L; (4) use according to any one of the aforementioned (1) to (3), wherein a concentration of ascorbic acid or the salt of ascorbic acid is 30 to 6000 mg/L; (5) use according to any one of the aforementioned (1) to (3), wherein a concentration of ascorbic acid or the salt of ascorbic acid is 300 to 3000 mg/L; (6) use according to any one of the aforementioned (1) to (5), wherein the platelet is a purified platelet obtained by a method comprising the following (A) and (B) : (A) a condensation step of condensing a culture product of megakaryocytes; and (B) a centrifugation step of centrifuging a platelet from the obtained condensed product; (7) use according to any one of the aforementioned (1) to (6), wherein the preservation solution further comprises albumin; (8) use according to the aforementioned (7), wherein a concentration of albumin is 1.25 to 10% (w/v); (9) use according to any one of the aforementioned (1) to (8), wherein the preservation solution further comprises sugar; (10) use according to the aforementioned (9), wherein the sugar is glucose; and (11) use according to any one of the aforementioned (1) to (10), for preserving a platelet or a megakaryocyte for 5 to 10 days.

The present invention also relates to: (12) use of a preservation solution for preserving a stem cell or an immunocyte for 1 to 63 days at 0 to 40°C, wherein the preservation solution comprises 10 to 5000 mg/L of niacin or a salt of niacin, 10 to 8000 mg/L of ascorbic acid or a salt of ascorbic acid, and trehalose in an isotonic solution; (13) use according to the aforementioned (12), wherein the stem cell is a mesenchymal stem cell; (14) use according to the aforementioned (12), wherein the immunocyte is a T cell; (15) use according to any one of the aforementioned (12) to (14), wherein the isotonic solution is a lactated Ringer's solution; (16) use according to any one of the aforementioned (12) to (15), wherein the isotonic solution further comprises dextran.

**Effect of the Invention**

According to the present invention, decrease in the functions and viability of mammalian cells during preservation can be efficiently suppressed as compared with a conventional preservation solution. Specifically, the aforementioned "mammalian cells" refers to platelets, megakaryocytes, stem cells and immunocytes. Hence, the present invention enables the targeted mammalian cells, which are difficult to freeze-preserve, to be preserved over a long period of time, and can provide a cell-containing liquid for transplantation having good quality in medical practice.

**Brief Description of Drawings**

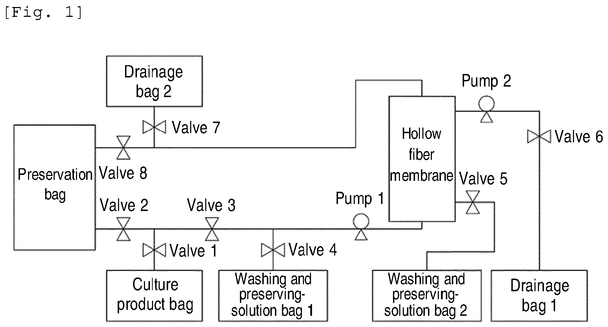

[Figure 1] Figure 1 is a schematic view showing a condensation system of iPS cell-derived platelets used in Examples of the present application.

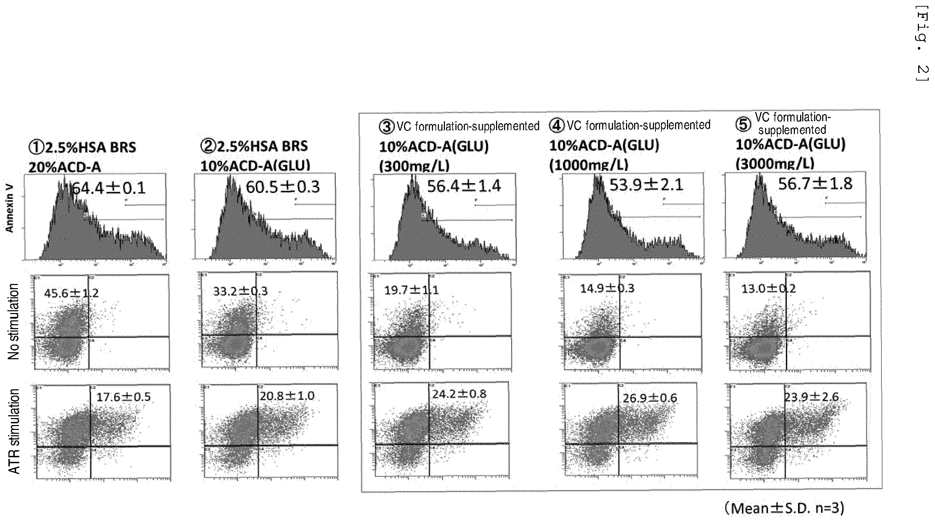

[Figure 2] Figure 2 is a diagram showing the influence of the preservation solution of the present invention on the Annexin V positive ratio, P-selectin positive ratio in the absence of stimulation, and PAC-1/P-selectin positive ratio in the presence of ATR stimulation of platelets after preservation for 5 days. In the diagram, "2.5%HSA BRS 20%ACD-A" and "2.5%HSA BRS 10%ACD-A(GLU)" each depict a conventional preservation solution; and "VC formulation-supplemented 10%ACD-A(GLU) (300 mg/L)", "VC formulation-supplemented 10%ACD-A(GLU) (1000 mg/L)", and "VC formulation-supplemented 10%ACD-A(GLU) (3000 mg/L)" each depict the preservation solution of the present invention containing 300 to 3000 mg/L VC.

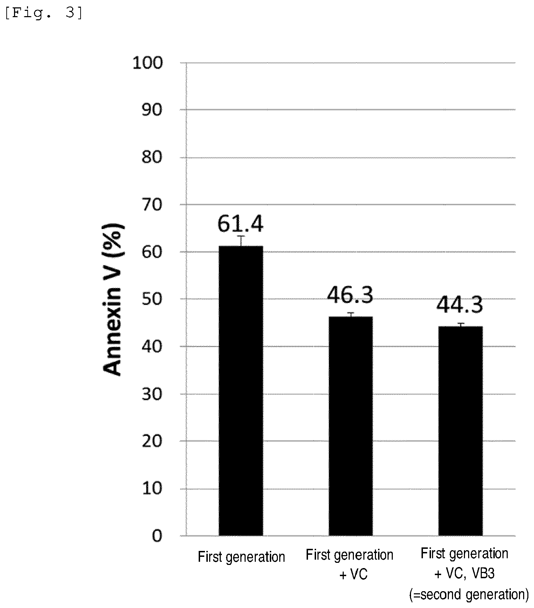

[Figure 3] Figure 3 is a diagram showing the influence of the preservation solution of the present invention on the Annexin V positive ratio of platelets after preservation for 5 days. In the diagram, "First generation" depicts a conventional preservation solution; "First generation+VC" depicts the preservation solution of the present invention containing 1000 mg/L VC; and "First generation+VC,VB3(=second generation)" depicts the preservation solution of the present invention containing 1000 mg/L VC and 400 mg/L nicotinic acid.

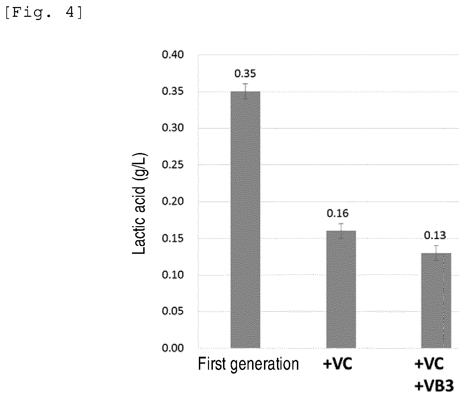

[Figure 4] Figure 4 is a diagram showing the influence of the preservation solution of the present invention on the lactic acid production of platelets after preservation for 5 days. In the diagram, "First generation" depicts a conventional preservation solution; "First generation+VC" depicts the preservation solution of the present invention containing 1000 mg/L VC; and "First generation+VC,VB3(=second generation)" depicts the preservation solution of the present invention containing 1000 mg/L VC and 400 mg/L nicotinic acid.

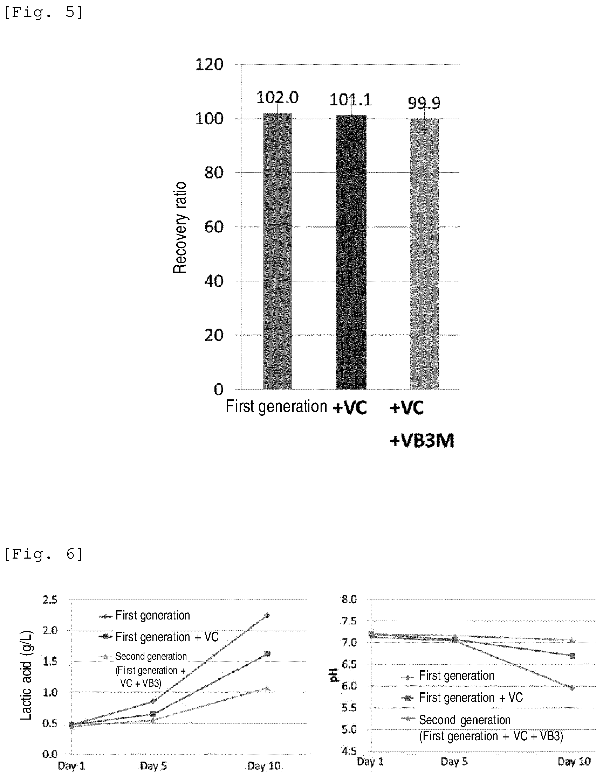

[Figure 5] Figure 5 is a diagram showing the influence of a preservation solution supplemented with VC and/or VB3 (nicotinic acid) on the recovery ratio of platelets after preservation for 5 days. In the diagram, "First generation" depicts a conventional preservation solution; "+VC" depicts the preservation solution of the present invention containing 1000 mg/L VC; and "+VC,VB3M" depicts the preservation solution of the present invention containing 1000 mg/L VC and 400 mg/L nicotinic acid.

[Figure 6] Figure 6 is a diagram showing the influence of the preservation solution of the present invention on the lactic acid concentration and pH of a platelet sample after preservation for 5 or 10 days. In the diagram, "First generation" depicts a conventional preservation solution; "First generation+VC" depicts the preservation solution of the present invention containing 1000 mg/L VC; and "Second generation (first generation+VC+VB3)" depicts the preservation solution of the present invention containing 1000 mg/L VC and 400 mg/L nicotinic acid.

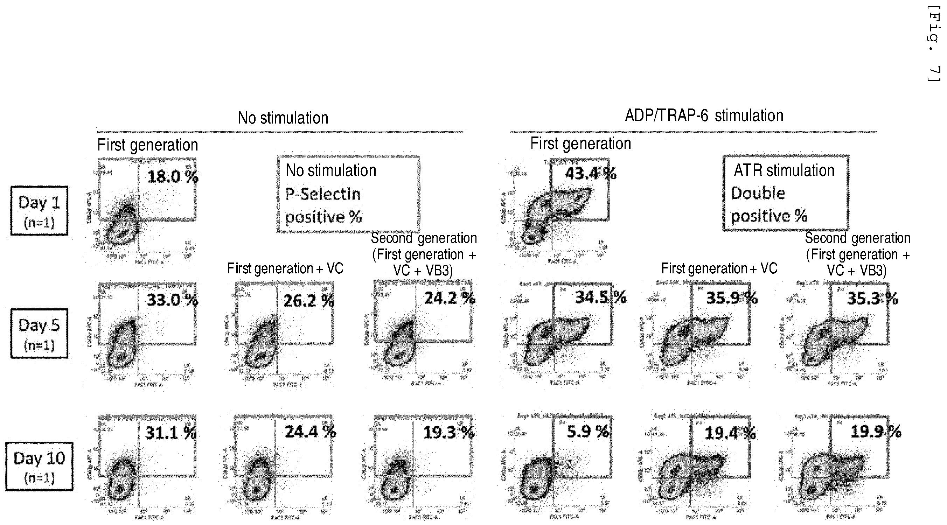

[Figure 7] Figure 7 is a diagram showing the influence of the preservation solution of the present invention on the Annexin V positive ratio, P-selectin positive ratio in the absence of stimulation, and PAC-1/P-selectin positive ratio in the presence of ATR stimulation of platelets after preservation for 5 or 10 days. In the diagram, "First generation" depicts a conventional preservation solution; "First generation+VC" depicts the preservation solution of the present invention containing 1000 mg/L VC; and "Second generation (first generation+VC+VB3)" depicts the preservation solution of the present invention containing 1000 mg/L VC and 400 mg/L nicotinic acid.

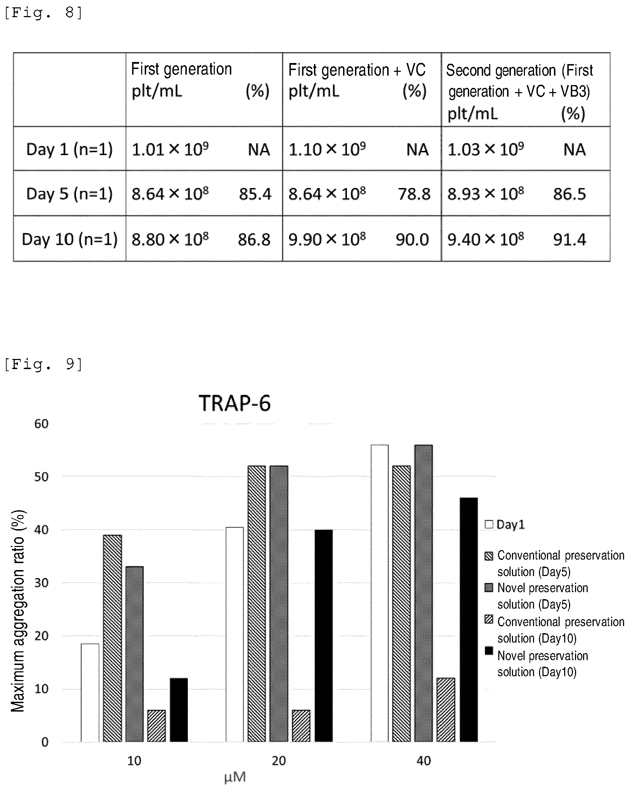

[Figure 8] Figure 8 is a diagram showing the influence of the preservation solution of the present invention on a platelet recovery ratio in a platelet sample after preservation for 5 or 10 days. In the diagram, "First generation" depicts a conventional preservation solution; "First generation+VC" depicts the preservation solution of the present invention containing 1000 mg/L VC; and "Second generation (first generation+VC+VB3)" depicts the preservation solution of the present invention containing 1000 mg/L VC and 400 mg/L nicotinic acid. In the diagram, "plt/mL" depicts a platelet concentration in the sample, and "%" depicts a platelet recovery ratio.

[Figure 9] Figure 9 is a diagram showing the influence of the preservation solution of the present invention on the aggregation capacity of platelets after preservation for 5 or 10 days. In this experiment, TRAP-6 was used as a stimulant. In the diagram, "Novel preservation solution" depicts the preservation solution of the present invention containing 1000 mg/L VC and 400 mg/L nicotinic acid.

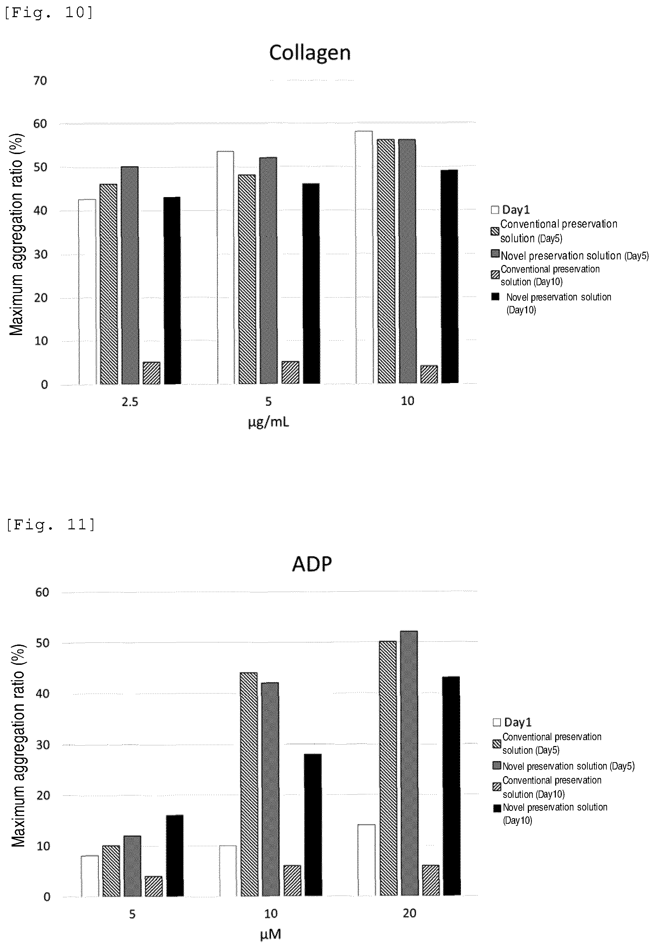

[Figure 10] Figure 10 is a diagram showing the influence of the preservation solution of the present invention on the aggregation capacity of platelets after preservation for 5 or 10 days. In this experiment, collagen was used as a stimulant. In the diagram, "Novel preservation solution" depicts the preservation solution of the present invention containing 1000 mg/L VC and 400 mg/L nicotinic acid.

[Figure 11] Figure 11 is a diagram showing the influence of the preservation solution of the present invention on the aggregation capacity of platelets after preservation for 5 or 10 days. In this experiment, ADP was used as a stimulant. In the diagram, "Novel preservation solution" depicts the preservation solution of the present invention containing 1000 mg/L VC and 400 mg/L nicotinic acid.

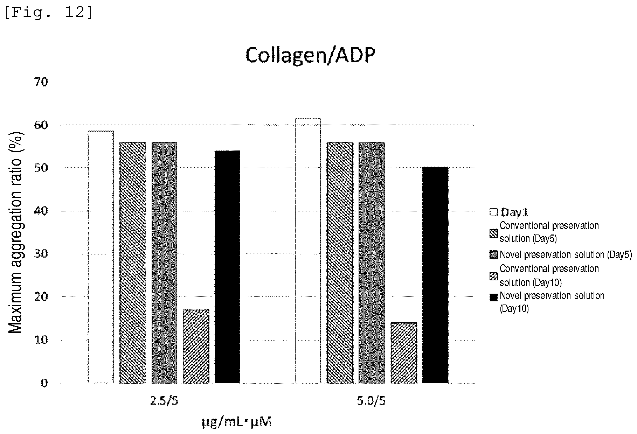

[Figure 12] Figure 12 is a diagram showing the influence of the preservation solution of the present invention on the aggregation capacity of platelets after preservation for 5 or 10 days. In this experiment, collagen/ADP was used as a stimulant. In the diagram, "Novel preservation solution" depicts the preservation solution of the present invention containing 1000 mg/L VC and 400 mg/L nicotinic acid.

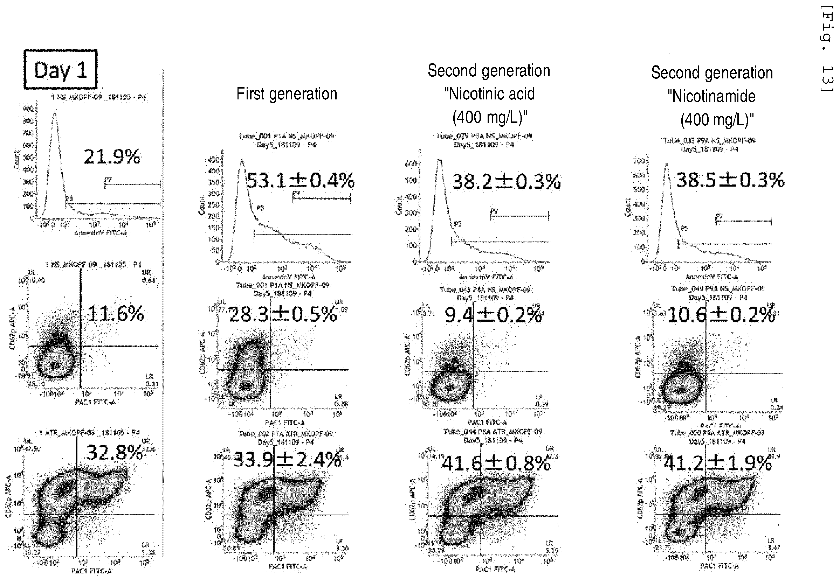

[Figure 13] Figure 13 is a diagram showing the influence of the preservation solution of the present invention on the Annexin V positive ratio, P-selectin positive ratio in the absence of stimulation, and PAC-1/P-selectin positive ratio in the presence of ATR stimulation of platelets after preservation for 5 days. In the diagram, "First generation" depicts a conventional preservation solution; "Second generation (nicotinic acid (400 mg/L)" depicts the preservation solution of the present invention containing 1000 mg/L VC and 400 mg/L nicotinic acid; and "Second generation (nicotinamide (400 mg/L)" depicts the preservation solution of the present invention containing 1000 mg/L VC and 400 mg/L nicotinamide.

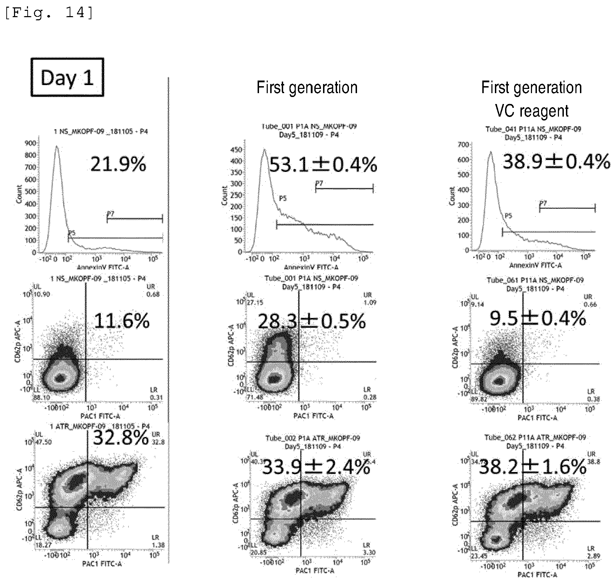

[Figure 14] Figure 14 is a diagram showing the influence of the preservation solution of the present invention on the Annexin V positive ratio, P-selectin positive ratio in the absence of stimulation, and PAC-1/P-selectin positive ratio in the presence of ATR stimulation of platelets after preservation for 5 days. In this experiment, an additive-free VC reagent was used. In the diagram, "First generation" depicts a conventional preservation solution; and "First generation VC reagent" depicts the preservation solution of the present invention containing 1000 mg/L VC.

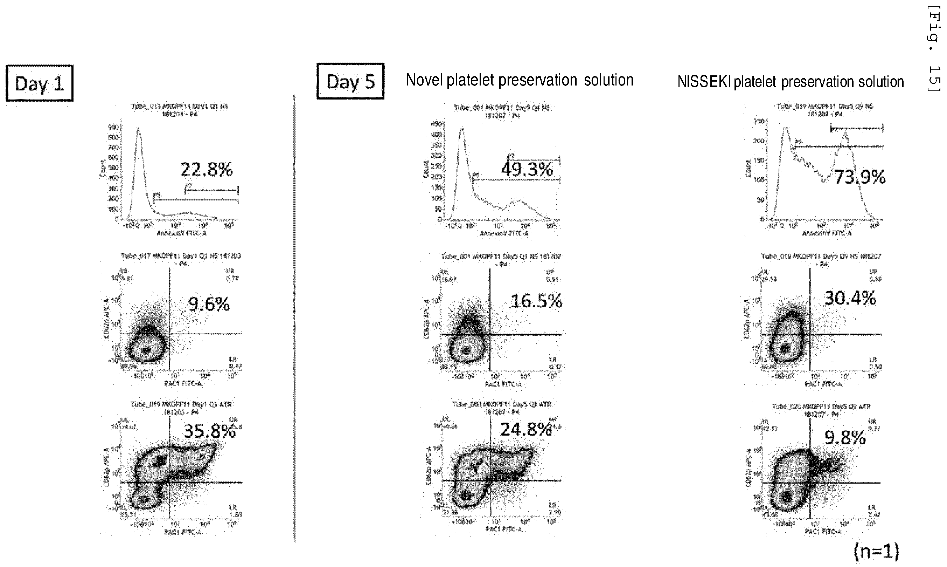

[Figure 15] Figure 15 is a diagram showing results of comparing the influence of the preservation solution of the present invention and a known preservation solution on the Annexin V positive ratio, P-selectin positive ratio in the absence of stimulation, and PAC-1/P-selectin positive ratio in the presence of ATR stimulation of platelets after preservation for 5 days. In the diagram, "Novel platelet preservations solution" depicts the preservation solution of the present invention containing 1000 mg/L VC and 400 mg/L nicotinic acid; and "NISSEKI platelet preservation solution" depicts a preservation solution containing an ACD-A solution and a bicarbonate Ringer's solution mixed at approximately 1:20.

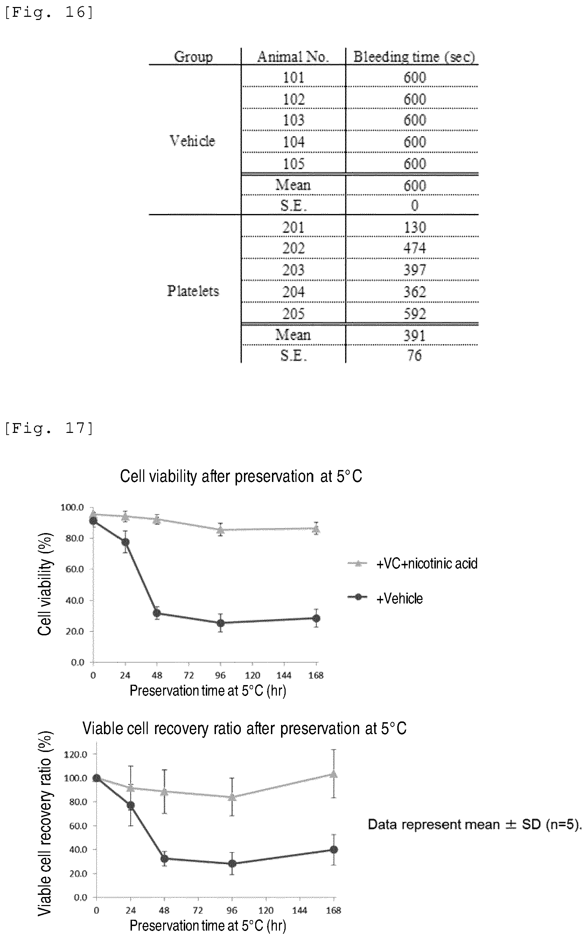

[Figure 16] Figure 16 is a diagram showing the hemostatic effect of a platelet sample prepared using the preservation solution of the present invention. In the diagram, "Vehicle" depicts mice given only the preservation solution of the present invention (free from platelets); and "Platelets" depicts mice given the platelet sample prepared using the preservation solution of the present invention.

[Figure 17] Figure 17 is a diagram showing the influence of the preservation solution of the present invention on the viability and viable cell recovery ratio of mesenchymal stem cells. In the diagram, "+Vehicle" depicts a preservation solution containing a CSP-01 solution supplemented with water; and "+VC+nicotinic acid" depicts the preservation solution of the present invention containing 1000 mg/L VC and 400 mg/L nicotinic acid in a CSP-01 solution.

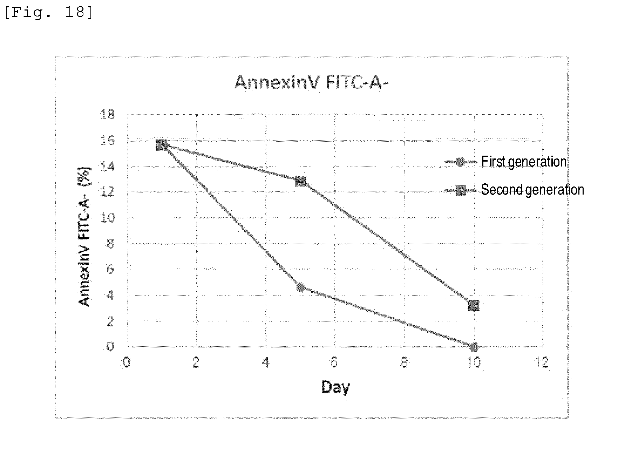

[Figure 18] Figure 18 is a diagram showing the influence of the preservation solution of the present invention on the Annexin V negative ratio of megakaryocytes. In the diagram, "First generation" depicts a conventional preservation solution; and "Second generation" depicts the preservation solution of the present invention containing 1000 mg/L VC and 400 mg/L nicotinic acid.

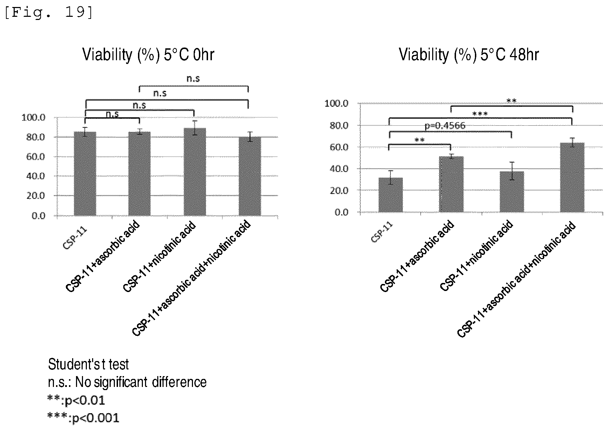

[Figure 19] Figure 19 is a diagram showing the influence of the preservation solution of the present invention on the viability and viable cell recovery ratio of T cells after preservation for 48 hours.

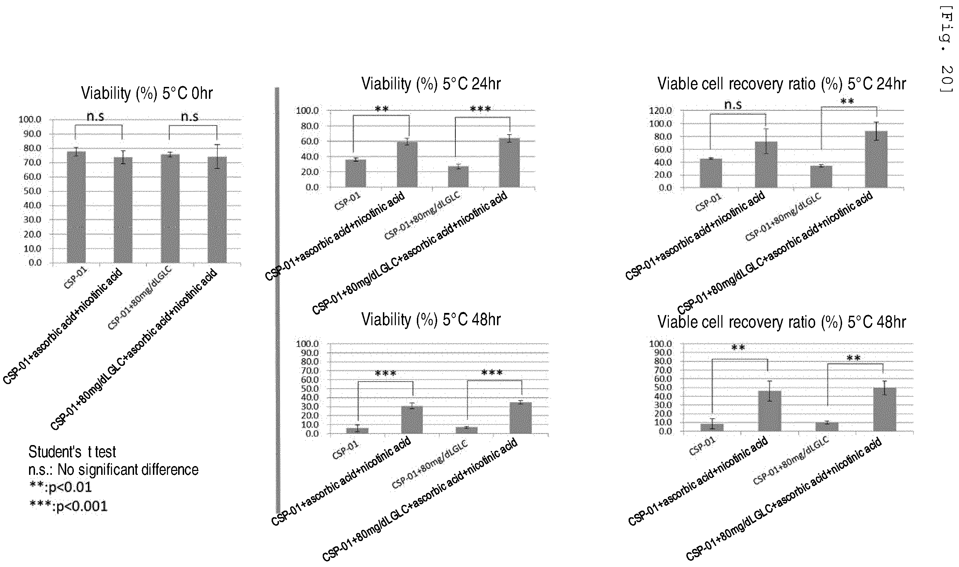

[Figure 20] Figure 20 is a diagram showing the influence of the preservation solution of the present invention on the viability and viable cell recovery ratio of T cells after preservation for 24 or 48 hours.

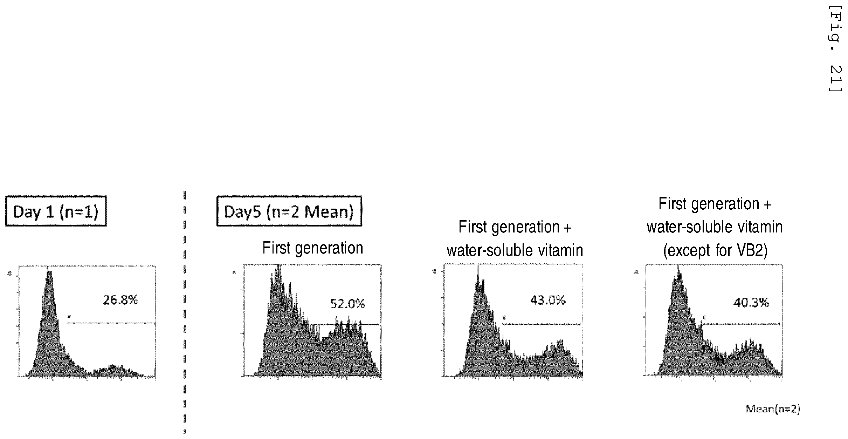

[Figure 21] Figure 21 is a diagram showing the influence of VB2 on the Annexin V positive ratio of platelets. In the diagram, "First generation" depicts a conventional preservation solution; "First generation+water-soluble vitamin" depicts the preservation solution of the present invention containing nine water-soluble vitamins including VB2; and "First generation+water-soluble vitamin (except for VB2)" depicts the preservation solution of the present invention containing eight water-soluble vitamins excluding VB2.

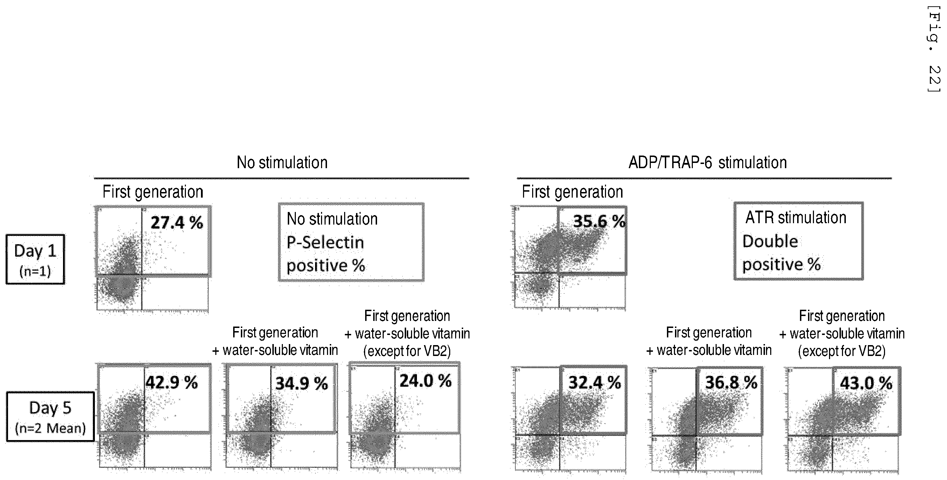

[Figure 22] Figure 22 is a diagram showing the influence of VB2 on the P-selectin positive ratio in the absence of stimulation, and PAC-1/P-selectin positive ratio in the presence of ATR stimulation of platelets. In the diagram, "First generation" depicts a conventional preservation solution; "First generation+water-soluble vitamin" depicts the preservation solution of the present invention containing nine water-soluble vitamins including VB2; and "First generation+water-soluble vitamin (except for VB2)" depicts the preservation solution of the present invention containing eight water-soluble vitamins excluding VB2.

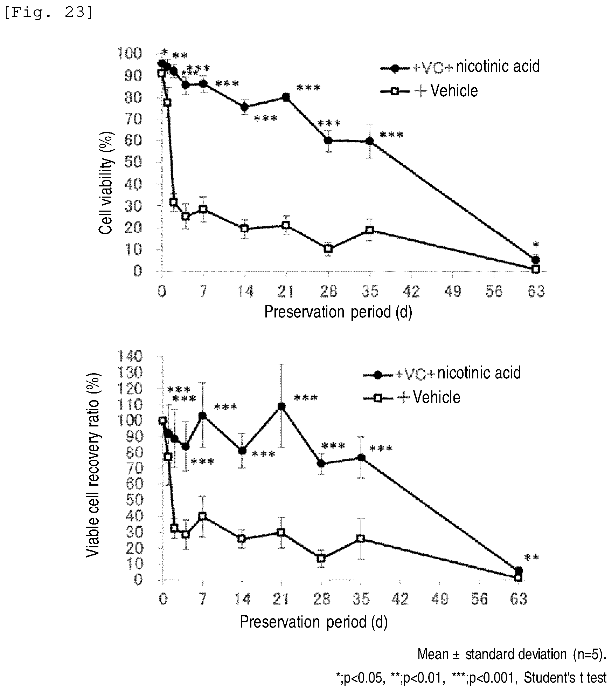

[Figure 23] Figure 23 is a diagram showing the influence of the preservation solution of the present invention on the viability and viable cell recovery ratio of mesenchymal stem cells. In the diagram, "+Vehicle" depicts a preservation solution containing a CSP-01 solution supplemented with water; and "+VC+nicotinic acid" depicts the preservation solution of the present invention containing a CSP-01 solution supplemented with 1000 mg/L VC and 400 mg/L nicotinic acid.

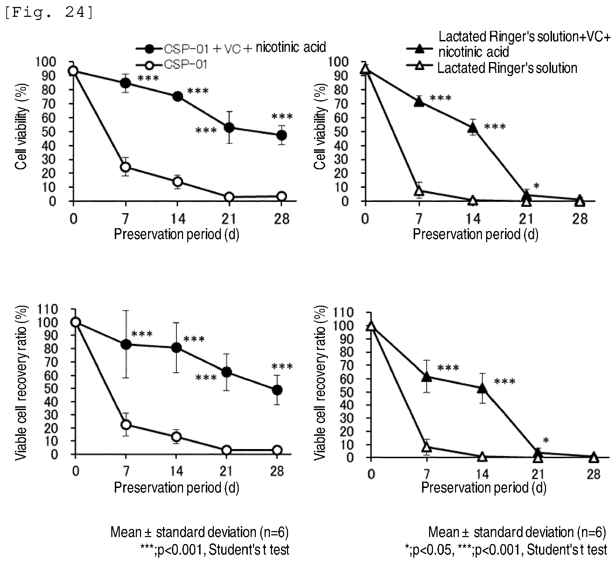

[Figure 24] Figure 24 is a diagram showing the influence of the preservation solution of the present invention on the viability and viable cell recovery ratio of mesenchymal stem cells. In the diagram, "CSP-01+VC+nicotinic acid" depicts the preservation solution of the present invention containing a CSP-01 solution supplemented with 1000 mg/L VC and 400 mg/L nicotinic acid; and "Lactated Ringer's solution+VC+nicotinic acid" depicts the preservation solution of the present invention containing a lactated Ringer's solution supplemented with 1000 mg/L VC and 400 mg/L nicotinic acid.

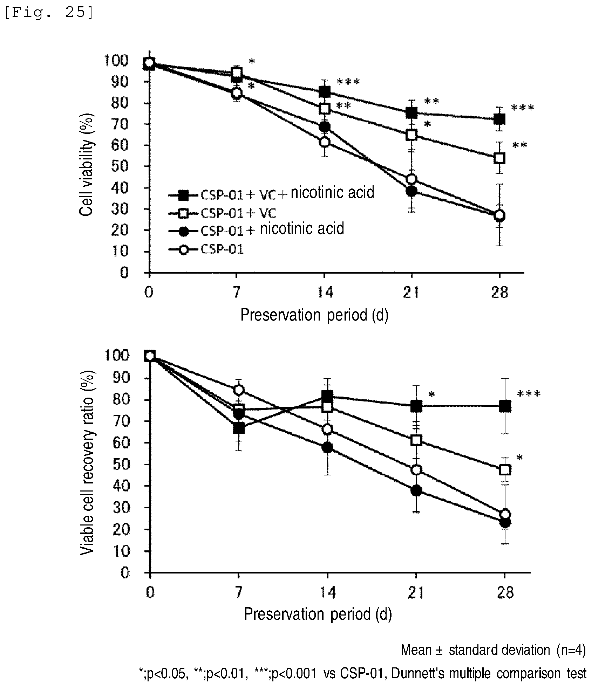

[Figure 25] Figure 25 is a diagram showing the influence of the preservation solution of the present invention on the viability and viable cell recovery ratio of mesenchymal stem cells. In the diagram, "CSP-01+VC+nicotinic acid" depicts the preservation solution of the present invention containing a CSP-01 solution supplemented with 1000 mg/L VC and 400 mg/L nicotinic acid; "CSP-01+VC" depicts the preservation solution of the present invention containing a CSP-01 solution supplemented with 1000 mg/L VC; and "CSP-01+nicotinic acid" depicts the preservation solution of the present invention containing a CSP-01 solution supplemented with 400 mg/L nicotinic acid.

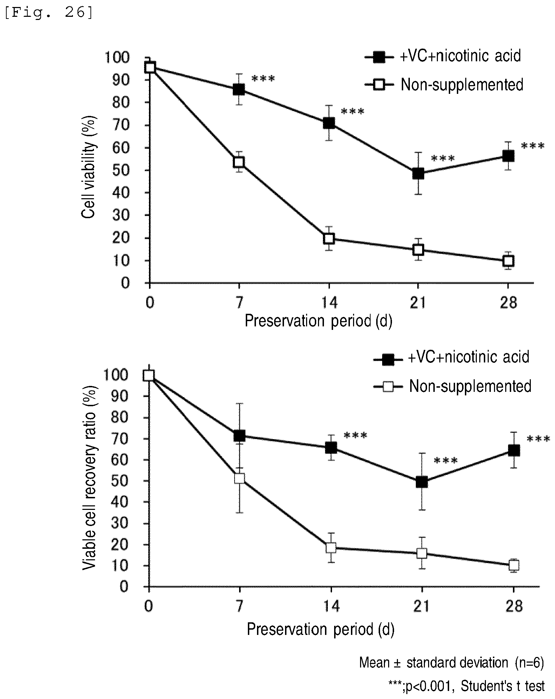

[Figure 26] Figure 26 is a diagram showing the influence of the preservation solution of the present invention on the viability and viable cell recovery ratio of young pig bone marrow mesenchymal stem cells. In the diagram, "Non-supplemented" depicts a CSP-01 solution; and "+VC+nicotinic acid" depicts the preservation solution of the present invention containing a CSP-01 solution supplemented with 1000 mg/L VC and 400 mg/L nicotinic acid.

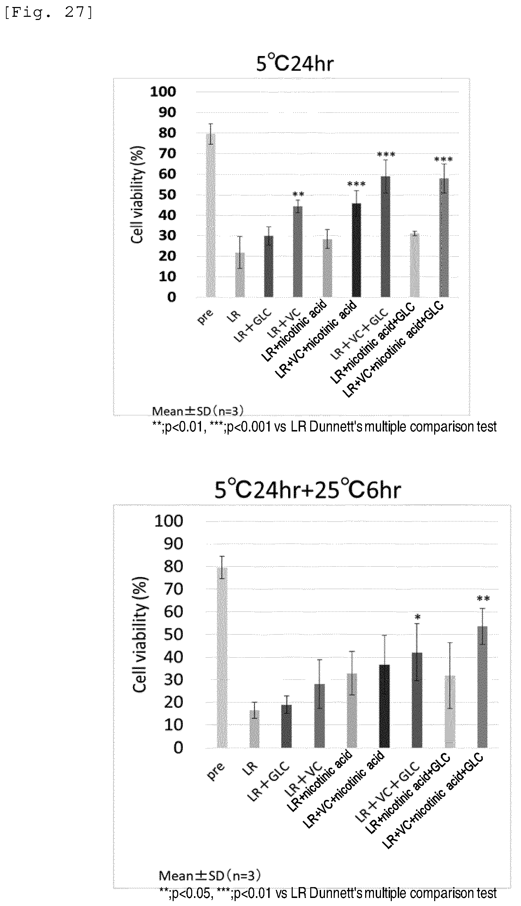

[Figure 27] Figure 27 is a diagram showing the influence of the preservation solution of the present invention on the viability of T cells. In the diagram, "Pre" depicts the viability of T cells before the start of preservation. In the diagram, "LR" depicts a lactated Ringer's solution; "GLC" depicts 80 mg/dL glucose; "VC" depicts 1000 mg/L VC; and nicotinic acid depicts 400 mg/L nicotinic acid.

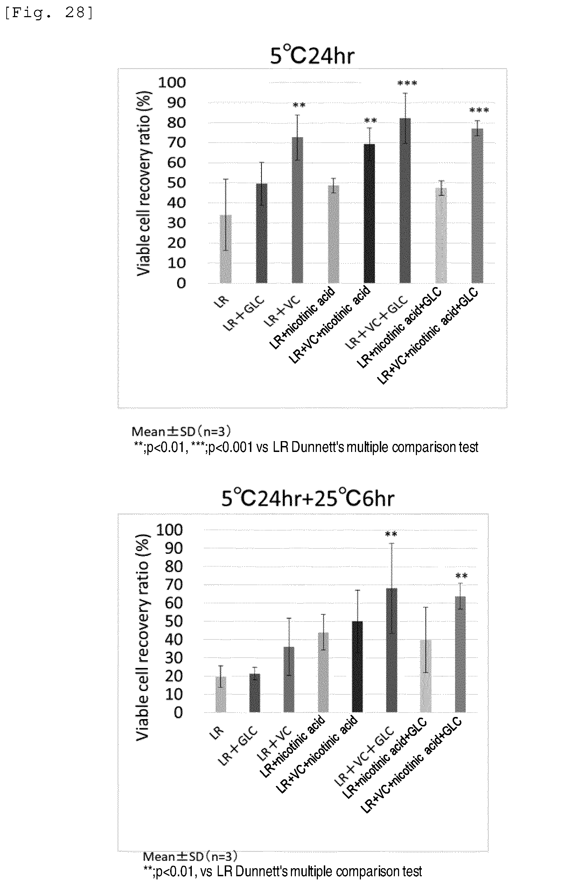

[Figure 28] Figure 28 is a diagram showing the influence of the preservation solution of the present invention on the viable cell recovery ratio of T cells. In the diagram, "LR" depicts a lactated Ringer's solution; "GLC" depicts 80 mg/dL glucose; "VC" depicts 1000 mg/L VC; and "nicotinic acid" depicts 400 mg/L nicotinic acid.

## Mode of Carrying Out the Invention

The use of a preservation solution for preserving a platelet or a megakaryocyte for 1 to 15 days at 0 to 40°C according to the present invention (hereinafter, also referred to as the "first aspect of the present invention") is not particularly limited so long as the preservation solution comprises niacin, etc. in an amount between 10 to 5000 mg/L and ascorbic acid, etc. in an amount of from 10 to 8000 mg/L, and does not comprise vitamin B2 or a salt of vitamin B2. The use of a preservation solution for preserving a stem cell or an immunocyte for 1 to 63 days at 0 to 40°C according to the present invention (hereinafter, also referred to as the "second aspect of the present invention") is not particularly limited so long as the preservation solution comprises 10 to 5000 mg/L of niacin, etc. and 10 to 8000 mg/L of ascorbic acid, etc. and trehalose in an isotonic solution. In this context, the phrase "preserving mammalian cells" means (i) maintaining survival, (ii) suppressing deterioration, (iii) maintaining a desired function, and/or (iv) suppressing differentiation or proliferation, for the targeted mammalian cells. Specifically, the preservation solution according to the first and second aspects of the present invention means a solution that exerts the aforementioned preservation effect in the case of being mixed with mammalian cells. The preservation solution of the present invention encompasses an embodiment of a preservation solution that has not yet contain cells to be preserved as well as an embodiment of a preservation solution already containing cells to be preserved. The preservation solution of the present invention according to both the first and second aspect of the invention comprises niacin, etc. and ascorbic acid, etc. in combination. Hereinafter, the niacin, etc. and the ascorbic acid, etc. is referred to as the "essential protective component of the present invention".

Niacin (VB3) in the aforementioned "niacin, etc." means nicotinic acid and/or nicotinamide. Specifically, the aforementioned preservation solution of the first and second aspect of the present invention can be any of a preservation solution comprising nicotinic acid, a preservation solution comprising nicotinamide, and a preservation solution comprising nicotinic acid and nicotinamide and is preferably a preservation solution comprising nicotinic acid. Niacin (VB3) can be manufactured by a known method based on chemical synthesis or the like, and a commercially available product may be used. Examples thereof can include commercially available products such as a nicotinic acid injection formulation (manufactured by TOA EIYO Ltd.) and nicotinamide (manufactured by FUJIFILM Wako Pure Chemical Corp.).

The present invention according to the first and second aspect may further comprise a derivative of niacin. Examples of the niacin derivative can suitably include, but are not particularly limited to, tocopherol nicotinic acid ester, niceritrol, nicomol, inositol hexanicotinic acid ester, 2-chloronicotinamide, 6-methylnicotinamide, 6-aminonicotinamide, N-methylnicotinamide, N,N-dimethylnicotinamide, N-(hydroxymethyl)nicotinamide, quinolinic acid imide, nicotinanilide, N-benzylnicotinamide, N-ethylnicotinamide, nifenazone, nicotinaldehyde, isonicotinic acid, methylisonicotinic acid, thionicotinamide, nialamide, 2-mercaptonicotinic acid, niaprazine, methyl nicotinate, and sodium nicotinate. Examples of the "salt of niacin or the derivative thereof" in the aforementioned "niacin, etc." can suitably include, but are not particularly limited to, alkali metal salts such as sodium salt and potassium salt, alkaline earth metal salts such as calcium salt and magnesium salt, organic base salts such as ammonium salt and trialkylamine salt, mineral acid salts such as hydrochloride and sulfate, and organic acid salts such as acetate.

The present invention according to the first and second aspect may further comprise an "antioxidant", but are not particularly limited to, superoxide dismutase 1, superoxide dismutase 2, superoxide dismutase 3, glutathione, lipoic acid, epigallocatechin gallate, curcumin, melatonin, hydroxytyrosol, ubiquinone, catalase, vitamin E, uric acid, their derivatives, and their salts. Ascorbic acid (VC) in the aforementioned "ascorbic acid, etc." can be manufactured by a known method based on chemical synthesis or the like, and a commercially available product may be used. Examples thereof can include commercially available products such as an ascorbic acid injection (manufactured by Sawai Pharmaceutical Co., Ltd.), and a L(+)-ascorbic acid standard (manufactured by FUJIFILM Wako Pure Chemical Corp.).

The present invention according to the first and second aspect may further comprise a derivative of ascorbic acid. Examples of the derivative of ascorbic acid (VC) can suitably include, but are not particularly limited to, ascorbic acid 2-phosphate, ascorbic acid 2-sulfate, ascorbyl-2-glucoside, and ascorbyl-6-glucoside. Examples of the salt of ascorbic acid (VC) in the aforementioned "ascorbic acid, etc." can suitably include, but are not particularly limited to, alkali metal salts such as sodium salt and potassium salt, alkaline earth metal salts such as calcium salt and magnesium salt, organic base salts such as ammonium salt and trialkylamine salt, mineral acid salts such as hydrochloride and sulfate, and organic acid salts such as acetate.

The concentration of the niacin, etc. contained in the preservation solution according to the first and second aspect of the present invention is in the range of 10 to 5000 mg/L based on nicotinic acid. Specifically, examples thereof can include 20 to 8000 mg/L, 30 to 6000 mg/L, 30 to 4000 mg/L, 30 to 3000 mg/L, 30 to 2000 mg/L, 30 to 1500 mg/L, and 30 to 1200 mg/L. Among them, the range of 120 to 1200 mg/L is preferred.

The concentration of the ascorbic acid, etc. contained in the preservation solution according to the first and second aspect of the present invention is in the range of 10 to 8000 mg/L based on ascorbic acid. Examples thereof can include 20 to 7000 mg/L, 30 to 6000 mg/L, 30 to 5000 mg/L, 30 to 4000 mg/L, 30 to 3000 mg/L, 50 to 3000 mg/L, and 100 to 3000 mg/L. Among them, the range of 300 to 3000 mg/L is preferred.

The preservation solution of the second aspect of the present invention comprises the essential protective component of the present invention in an isotonic solution. The preservation solution of the first aspect of the present invention may comprise the essential protective component of the present invention in an isotonic solution. The aforementioned "isotonic solution" is not particularly limited so long as the isotonic solution has a salt concentration or a sugar concentration, etc. adjusted with a sodium ion, a potassium ion, a calcium ion, or the like so as to be substantially the same as that of the osmotic pressure of body fluid or cell fluid. Specifically, examples thereof can include saline, saline having a buffering effect (phosphate buffered saline [PBS], Tris buffered saline [TBS], HEPES buffered saline, etc.), Ringer's solutions, lactated Ringer's solutions, acetated Ringer's solutions, and bicarbonate Ringer's solutions. Among them, a bicarbonate Ringer's solution or a lactated Ringer's solution is preferred. The isotonic solution can be manufactured on the basis of known makeup, and a commercially available product may be used. Examples of the commercially available one can include commercially available products such as Otsuka Normal Saline (isotonic sodium chloride solution, manufactured by Otsuka Pharmaceutical Factory), Ringer's Solution "Otsuka" (Ringer's solution, manufactured by Otsuka Pharmaceutical Factory), Lactec(R) Injection (lactated Ringer's solution, manufactured by Otsuka Pharmaceutical Factory), Veen(R) F Infusion Solution (acetated Ringer's solution, manufactured by Fuso Pharmaceutical Industries, Ltd.), and Bicanate(R) Infusion Solution (bicarbonate Ringer's solution, manufactured by Otsuka Pharmaceutical Factory). In the present specification, the term "isotonic" means that the osmotic pressure is in the range of 250 to 380 mOsm/L.

The preservation solution of the present invention may further comprise albumin or a sugar as an optional active ingredient for preserving the target mammalian cells. In the present specification, the "optional active ingredient" means a component that may or may not be contained. Albumin and/or the sugar is also referred to as the "optional protective component of the present invention". Examples of the aforementioned "albumin" can include human serum albumin (HSA), bovine serum albumin (BSA), and fetal bovine serum albumin (FBS). HSA is preferred. In the case where the aforementioned preservation solution of the present invention comprises albumin, the concentration of albumin can be in the range of 0.1 to 30 (w/v)%. Examples thereof can include 1.0 to 20 (w/v)%, 1.0 to 15 (w/v)%, 1.0 to 10 (w/v)%, 1.25 to 10 (w/v)%, 1.25 to 7.25 (w/v)%, 1.5 to 5.0 (w/v)%, 1.5 to 2.5 (w/v)%, and 2.0 to 2.5 (w/v)%.

Examples of the aforementioned "sugar" can include grape sugar (glucose), trehalose, dextran, and hydroxyethyl starch. In the case where the aforementioned preservation solution of the present invention comprises grape sugar, the concentration of grape sugar can be in the range of 1 to 10000 mg/L. Examples thereof can include 10 to 8000 mg/L, 20 to 6000 mg/L, 30 to 6000 mg/L, 40 to 6000 mg/L, 50 to 6000 mg/L, 100 to 6000 mg/L, 200 to 6000 mg/L, 500 to 6000 mg/L, 1000 to 6000 mg/L, 2000 to 6000 mg/L, and 2000 to 5000 mg/L. The preservation solution according to the second aspect of the invention comprises trehalose. The preservation according to the first aspect of the invention may comprise trehalose. Examples of the concentration of trehalose can include 0.1 to 100 g/L, 5 to 80 g/L, and 20 to 60 g/L. In the case where the aforementioned preservation solution of the present invention comprises dextran, examples of the concentration of dextran can include 0.1 to 100 g/L, 5 to 80 g/L, and 40 to 70 g/L. In the case where the aforementioned preservation solution of the present invention comprises hydroxyethyl starch, examples of the concentration of hydroxyethyl starch can include 1 to 500 g/L and 10 to 100 g/L.

The preservation solution of the first aspect does not comprise (a) vitamin B2 or a salt of vitamin B2. It is preferred that the preservation solution according to second aspect of the present invention should not comprise (a) vitamin B2 (also referred to as VB2 or riboflavin) or a derivative thereof or a salt of vitamin B2 or the derivative (hereinafter, also referred to as "vitamin B2, etc."). Further, it is preferred that the preservation solution according to both the first and second aspect of the present invention should not comprise (b) a culture medium or an essential component thereof, or (c) a cell differentiation enhancing agent. Examples of the derivative of vitamin B2 in the aforementioned "vitamin B2, etc." (a) can include flavin mononucleotide, flavin adenine dinucleotide, riboflavin tetrabutyrate, riboflavin butyrate, and riboflavin phosphate (riboflavin phosphoric acid ester). Examples of the salt of vitamin B2 or the derivative thereof can include sodium salt.

The aforementioned "culture medium" (b) means a solution for cell culture that provides nutrients necessary for the maintenance and proliferation of cells in an *in vitro* environment. Examples thereof can include Eagle's minimal essential (EME) medium, Iscove's modified Dulbecco's medium (IMDM), Dulbecco's modified Eagle medium (DMEM), TC199 medium, alpha minimal essential medium (α-MEM), RPMI1640, Ham-F-12, E199, MCDB, Leibovitz's L-15, and William's E medium. The aforementioned "essential component of the culture medium" (b) means an aqueous nutrient and electrolyte, glycosaminoglycan, a deswelling agent, an energy source, a buffer, an antioxidant, a membrane stabilizer, an antibiotic (or an antifungal agent), an ATP precursor, a cell nutrient supplement, and/or a pH indicator. Examples of the aforementioned "aqueous nutrient and electrolyte" can include one or more culture media selected from the aforementioned culture media. Examples of the aforementioned "glycosaminoglycan" can include chondroitin sulfate, dermatan sulfate, dermatin sulfate, heparin sulfate, heparan sulfate, keratin sulfate, keratan sulfate, and hyaluronic acid. Examples of the aforementioned "deswelling agent" can include dextran, dextran sulfate, polyvinylpyrrolidone, polyethylene glycol, polyvinyl acetate, hydroxypropylmethylcellulose, and carboxypropylmethylcellulose. Examples of the aforementioned "energy source" can include pyruvate, sucrose, fructose, and dextrose. Examples of the aforementioned "buffer" can include bicarbonate buffer solutions and HEPES buffer solutions. Examples of the aforementioned "antioxidant" can include 2-mercaptoethanol, glutathione, and $\alpha$-tocopherol. Examples of the aforementioned "membrane stabilizer" can include vitamin A, retinoic acid, ethanolamine, phosphoethanolamine, selenium, and transferrin. Examples of the aforementioned "antibiotic and/or antifungal agent" can include amphotericin-B, gentamicin sulfate, kanamycin sulfate, neomycin sulfate, nystatin, penicillin, tobramycin, and streptomycin. Examples of the aforementioned "ATP precursor" can include adenosine, inosine, and adenine. Examples of the aforementioned "cell nutrient supplement" can include cholesterol, L-hydroxyproline, d-biotin, calciferol, niacin, p-aminobenzoic acid, pyridoxin hydrochloride, vitamin B12, $Fe(NO_3)_3$, and nonessential amino acids. Examples of the aforementioned "pH indicator" can include phenol red.

The aforementioned "cell differentiation enhancing agent" (c) means a drug that is added to a culture medium or the like in order to obtain a desired type of cells from cells having the ability to differentiate. Examples thereof can include retinol, vitamin D2, vitamin D3, vitamin K, retinoic acid, zinc, zinc compounds, calcium, calcium compounds, hydrocortisone, dexamethasone, L-glutamine, ethylene glycol tetraacetate (EGTA), proline, nonessential amino acids (NEAA), $\beta$-mercaptoethanol, dibutyl cyclic adenosine monophosphate (db-cAMP), monothioglycerol (MTG), putrescine, dimethyl sulfoxide (DMSO), hypoxanthine, adenine, forskolin, cilostamide, 3-isobutyl-1-methylxanthine, 5-azacitidine, pyruvate, okadaic acid, linoleic acid, ethylenediaminetetraacetic acid (EDTA), an anticoagulant dextrose citrate A (ACDA), EDTA disodium, sodium butyrate, glycerophosphate, G418, gentamycin, pentoxifylline (1-(5-oxohexyl)-3,7-dimethylxanthine), indomethacin, and tissue plasminogen activator (TPA). As mentioned above, in the present invention, the niacin, etc., and the antioxidant (including the ascorbic acid, etc.) are used for preserving mammalian cells and are not used as a differentiation enhancing agent.

The "mammalian cells" targeted by the preservation solution according to the first aspect of the present invention are platelets for use in the treatment or the like of diseases or trauma, and megakaryocytes for use in the manufacture thereof. The "mammalian cells" targeted by the preservation solution according to the second aspect of the present invention are stem cells and immunocytes for use in regenerative medicine or immunotherapy. Examples of the aforementioned "mammal" can include rodents such as mice, rats, hamsters, and guinea pigs, lagomorphs such as rabbits, ungulates such as pigs, cattle, goats, horses, and sheep, carnivora such as dogs and cats, and primates such as humans, monkeys, rhesus macaques, cynomolguses, marmosets, orangutans, and chimpanzees. Among them, a human can be suitable.

The aforementioned "platelets" are one of the cellular components in blood and mean a cellular component positive to CD41a and CD42b. The platelets targeted by the preservation solution of the present invention may be condensed platelets obtained by removing erythrocytes and leukocytes from the blood (whole blood) of a mammal, or may be purified platelets artificially manufactured from megakaryocyte cultured *in vitro,* and are preferably purified platelets. Examples of the method for manufacturing the aforementioned purified platelets can suitably include, but are not particularly limited to, a method comprising the steps (A) and (B) : (A) a condensation step of condensing a culture product of megakaryocytes; and (B) a centrifugation step of centrifuging platelets from the obtained condensed product. The "megakaryocytes" that are also used in such a method for manufacturing the platelets can be induced from pluripotent cells such as induced pluripotent stem cells (iPS cells), embryonic stem cells (ES cells), non-human nuclear transfer ES cells (ntES cells), germinal stem cells (EG cells), somatic stem cells, or embryonal tumor cells, and can also be induced from hematopoietic stem cells isolated from bone marrow, umbilical cord blood, peripheral blood, or the like, hematopoietic progenitor cells, CD34-positive cells, megakaryocytes-erythroid progenitor cells, megakaryocyte progenitor cells, or the like.

The aforementioned "stem cells" mean immature cells having the ability to self-renew and the ability to differentiate and/or proliferate. The stem cells include subpopulations such as pluripotent stem cells, multipotent stem cells, and unipotent stem cells according to the ability to differentiate. The pluripotent stem cells mean cells that cannot become an individual in themselves, but are capable of differentiating into every tissue or cell constituting the living body. The multipotent stem cells mean cells that are capable of differentiating into plural types, not all types, of tissues or cells. The unipotent stem cells mean cells that are capable of differentiating into a particular tissue or cell. The stem cells to be preserved in the preservation solution of the present invention can be any of pluripotent stem cells, multipotent stem cells, and unipotent stem cells. Examples thereof can include pluripotent stem cells such as iPS cells, ES cells, non-human ntES cells, and EG cells, and somatic stem cells such as mesenchymal stem cells, hematopoietic stem cells, neural stem cells, bone marrow stem cells, and germ stem cells. Among them, mesenchymal stem cells are preferred. The mesenchymal stem cells can be collected by a publicly known general method from the bone marrow, fat tissue, peripheral blood, umbilical cord blood, etc. of a mammal. In addition, human mesenchymal stem cells can be isolated by the culture or subculture of hematopoietic stem cells or the like after bone marrow puncture. Among them, examples thereof can suitably include mesenchymal stem cells derived from human bone marrow, and mesenchymal stem cells derived from young pig bone marrow.

The aforementioned "immunocytes" mean cells that are present in blood or lymph and are involved in the immune system. Examples of the immunocytes to be preserved in the preservation solution of the present invention can include T cells, macrophages, dendritic cells, B cells, NK cells, neutrophils, eosinophils, and myeloid-derived suppressor cells (MDSC). Among them, T cells are preferred. The "T cells" mean cells expressing an antigen receptor called T cell receptor (TCR) on the surface. Examples thereof include cytotoxic T cells which are CD8-positive cells, helper/regulatory T cells which are CD4-positive cells, naive T cells (CD45RA+CD62L+ cells), central memory T cells (CD45RA-CD62L+ cells), effector memory T cells (CD45RA-CD62L- cells), and terminal effector T cells (CD45RA+CD62L-cells). The T cells can be collected by a publicly known general method from the peripheral blood, lymph node, bone marrow, thymus gland, spleen, umbilical cord blood, etc. of a mammal. In addition, a commercially available product may be used.

The preservation solution of the present invention can be used for preserving mammalian cells in a non-frozen state. The temperature in preserving mammalian cells by use of the present invention is 0 to 40°C. For example, the temperature in preserving platelets or megakaryocytes by use of the preservation solution according to the first aspect of the present invention is preferably 15 to 30°C, more preferably 20 to 24°C, most preferably 21 to 23°C. The temperature in preserving mesenchymal stem cells or T cells by use of the preservation solution according to the second aspect of the present invention is preferably 1 to 38°C, more preferably 1 to 30°C, particularly preferably 1 to 15°C, most preferably 1 to 5°C. The temperature in preserving mammalian cells by use of the present invention may be changed within the range of 0 to 40°C. For example, as shown in Examples given below, in preserving T cells by use of the preservation solution of the present invention, the T cells can be preserved at a low temperature (e.g., 1 to 5°C, preferably 5°C) for a given period and then further preserved at room temperature (e.g., 20 to 26°C, preferably 22 to 25°C, more preferably 25°C) for several hours. Also, the preservation solution of the present invention is used for preserving mammalian cells for several hours to several tens of days. Although the preservation period differs depending on the type of the cells to be preserved, as shown in Examples given below. According to the first aspect of the present invention, platelets or megakaryocytes are preserved for 1 to 15 days, preferably 1 to 10 days. According to the second aspect of the present invention are preserved for 1 to 63 days. Mesenchymal stem cells can be preserved for 1 to 63 days, preferably 1 to 35 days, more preferably 1 to 30 days, still more preferably 1 to 28 days, further preferably 1 to 14 days, and T cells can be preserved for 1 to 30 days, preferably 1 to 14 days, more preferably 1 to 2 days, further preferably 30 hours.

Use of the preservation solution of the present invention may be used for the purpose of administering mammalian cells to a mammal. Specifically, a mixed solution containing the preservation solution of the present invention and mammalian cells is preserved under predetermined conditions. Then, the mixed solution may be administered (e.g., intravenously administered) as such into the living body of a mammal. Hence, it is preferred that the preservation solution according to the first and second aspects of the present invention should not comprise a component that may have an adverse effect on the living body of a mammal when administered into the living body of the mammal. Examples of such a "component that may have an adverse effect" can include polyvinylpyrrolidone, 2-mercaptoethanol, okadaic acid, sodium butyrate, and G418.

Use of a preservation solution according to the present invention can be for a deterioration suppressor for mammals. When using the preservation solution according to the present invention as a deterioration suppressor, the preservation solution may further comprise the optional protective component of the present invention. Use of the preservation solution according to the present invention as a deterioration suppressor for mammals of the present invention can be used for preparing the preservation solution of the present invention by addition to the aforementioned isotonic solution, and additionally, can be used for enhancing the preservation stability of a known preservation solution for mammals by addition to the preservation solution. Use of a preservation solution according to of the present invention can be of in a mammalian cell preservation container in which the preservation solution of the present invention is enclosed. The preservation container of the present invention can be in any form so long as the container can keep sterility after injection of a liquid suspension of mammalian cells. Examples thereof can include blood bags, infusion solution bags, syringes, ampules, and vials. A blood bag is preferred.

Hereinafter, the present invention will be more specifically described with reference to Examples. However, the technical scope of the present invention is in no way limited by these examples.

Example 1

[Preparation of iPS cell-derived platelets]

iPS cell-derived platelets were prepared according to the method described in PCT/JP2018/034667. Specific procedures will be shown in the following (1-1) to (1-12).

(1-1) Preparation of hematopoietic progenitor cells form iPS cells

Differentiation culture from human iPS cells (TKDN SeV2 and NIH5: human embryonic skin fibroblast-derived iPS cells established using hemagglutinating virus of Japan) into blood cells was carried out according to the method of Takayama et al. (J. Exp. Med., 2010, vol. 13, 2817-2830). Specifically, a human ES/iPS cell colony was cocultured with C3H10T1/2 feeder cells for 14 days in the presence of 20 ng/mL VEGF (manufactured by R&D Systems, Inc.) to prepare hematopoietic progenitor cells (HPC). The aforementioned culture was carried out under conditions of 37°C, 20% $O_2$, and 5% $CO_2$.

(1-2) Gene transfer system

The gene transfer system used was a lentivirus vector system. The lentivirus vector is tetracycline-regulated Tet-on(R) gene expression induction system vector. The vector was prepared by recombining the mOKS cassette of LV-TRE-mOKS-Ubc-tTA-I2G (Kobayashi et al., Cell, 2010, vol. 142, No. 5, 787-799) with c-MYC, BMI1, or BCL-xL. The vector harboring c-MYC, BMI1, or BCL-xL was designated as LV-TRE-c-Myc-Ubc-tTA-I2G, LVTRE-BMI1-Ubc-tTA-I2G, and LV-TRE-BCL-xL-Ubc-tTA-I2G, respectively. c-MYC, BMI1, and BCL-xL viruses were prepared by gene transfer into 293T cells through the aforementioned lentivirus vector. The target cells were infected with the obtained viruses so that the c-MYC, BMI1, and BCL-xL genes were transferred to the genomic sequences of the target cells. These genes stably transferred into the genomic sequences can be forcedly expressed by the addition of doxycycline (Clontech #631311) to a culture medium.

(1-3) Infection of hematopoietic progenitor cells with c-MYC and BMI1 viruses

A 6-well plate inoculated with C3H10T1/2 feeder cells in advance was inoculated with $5 \times 10^4$ cells/well of HPC obtained by the method of the aforementioned (1-1), and c-MYC and BMI1 were forcedly expressed by the lentivirus method using the BMI1 virus and the c-MYC virus. In this respect, 6 wells were used per type of cell line. Specifically, particles of each virus were added at MOI (multiplicity of infection) of 20 into a culture medium, and the cells were infected therewith by spin infection (32°C, 900 rpm, 60 min, centrifugation). The aforementioned spin infection was carried out twice at an interval of 12 hours. The culture medium used was a basal medium (IMDM (manufactured by Sigma-Aldrich Co. LLC) containing 15% Fetal Bovine Serum (manufactured by Gibco/Thermo Fisher Scientific Inc.), 1% Penicillin-Streptomycin-Glutamine (manufactured by Gibco/Thermo Fisher Scientific Inc.), 1% Insulin, Transferrin, Selenium Solution (ITS-G) (manufactured by Gibco/Thermo Fisher Scientific Inc.), 0.45 mmol/L 1-Thioglycerol (manufactured by Sigma-Aldrich Co. LLC), and 50 µg/mL L-Ascorbic Acid (manufactured by Sigma-Aldrich Co. LLC)) supplemented with 50 ng/mL Human thrombopoietin (TPO) (manufactured by R&D Systems, Inc.), 50 ng/mL Human Stem Cell Factor (SCF) (manufactured by R&D Systems, Inc.) and 2 µg/mL Doxycycline (DOX, manufactured by Clontech Laboratories, Inc., #631311) (hereinafter, this culture medium is referred to as a "differentiation culture medium") and further supplemented with protamine (final concentration: 10 µg/mL).

(1-4) Preparation and maintenance culture of self-proliferating megakaryocyte line

The day on which the infection with the c-MYC and BMI1 viruses was carried out by the method of the aforementioned (1-3) was defined as infection day 0. HPC harboring the c-MYC gene and the BMI1 gene was cultured as mentioned below to prepare each self-proliferating megakaryocyte line. The forced expression of the c-MYC gene and the BMI1 gene was carried out by the addition of 1 µg/mL DOX to a culture medium.

Infection day 2 to infection day 11:

On infection day 2, the virus-infected blood cells obtained by the aforementioned method were recovered by pipetting, and centrifugal operation was performed at 1200 rpm for 5 minutes to remove a supernatant. Then, the cells were suspended in a fresh differentiation culture medium and inoculated onto fresh C3H10T1/2 feeder cells (6-well plate). On infection day 9, subculture was carried out by the same operation as above. At the time of the aforementioned reinoculation, a cell number was counted, and the cells were then inoculated at $1 \times 10^5$ cells/2 mL/well onto the C3H10T1/2 feeder cells (6-well plate). Infection day 12 to infection day 13:

The same operation as in infection day 2 was carried out. A cell number was counted, and the cells were then inoculated at $3 \times 10^5$ cells/10 mL/100 mm dish onto C3H10T1/2 feeder cells (100 mm dish).

Infection day 14:

The virus-infected blood cells were recovered, and the aforementioned blood cells were reacted with each antibody using 2 µL of anti-human CD41a-APC antibody (manufactured by BioLegend, Inc.), 1 µL of anti-human CD42b-PE antibody (manufactured by eBioscience, Inc.), and 1 µL of anti-human CD235ab-pacific blue antibody (manufactured by BioLegend, Inc.) per $1.0 \times 10^5$ cells. The cells thus reacted were analyzed using FACS Verse(TM) (manufactured by BD Biosciences). On infection day 14, cells having a CD41a positive ratio of 50% or more were regarded as a self-proliferating megakaryocyte line.

(1-5) Infection of self-proliferating megakaryocyte line with BCL-xL virus

The BCL-xL gene was transferred to the aforementioned self-proliferating megakaryocyte line of infection day 14 by the lentivirus method using the BCL-xL virus. Particles of the virus were added at MOI of 10 into a culture medium, and the cells were infected therewith by spin infection (32°C, 900 rpm, 60 min, centrifugation). The forced expression of the BCL-xL gene was carried out by the addition of DOX at 1 $\mu$g/mL DOX to the culture medium.

(1-6) Preparation and maintenance culture of immortalized megakaryocyte line

Infection day 14 to infection day 18:

The self-proliferating megakaryocyte line harboring the BCL-xL gene obtained by the method of the aforementioned (1-5) was recovered, and centrifugal operation was performed at 1200 rpm for 5 minutes. Cells precipitated by the aforementioned centrifugation were suspended in a fresh differentiation culture medium and then inoculated at $2 \times 10^5$ cells/2 mL/well onto fresh C3H10T1/2 feeder cells (6-well plate).

Infection day 18 (subculture):

The self-proliferating megakaryocyte line harboring the BCL-xL gene was recovered. A cell number was counted, and the cells were then inoculated at $3 \times 10^5$ cells/10 mL/100 mm dish.

Infection day 24 (subculture):

The self-proliferating megakaryocyte line harboring the BCL-xL gene was recovered. A cell number was counted, and the cells were then inoculated at $1 \times 10^5$ cells/10 mL/100 mm dish. Thereafter, subculture was performed in the same manner as above every 4 to 7 days for maintenance culture. At the time of the subculture, the cells were suspended in a fresh differentiation culture medium and then inoculated.

On infection day 24, the self-proliferating megakaryocyte line harboring the transferred BCL-xL gene was recovered and immunostained with 2 $\mu$L of anti-human CD41a-APC antibody (manufactured by BioLegend, Inc.), 1 $\mu$L of anti-human CD42b-PE antibody (manufactured by eBioscience, Inc.), and 1 $\mu$L of anti-human CD235ab-Pacific Blue antibody (Anti-CD235ab-PB; manufactured by BioLegend, Inc.) per $1.0 \times 10^5$ cells, followed by analysis using FACS Verse(TM). On infection day 24, a line having a CD41a positive ratio of 50% or more was regarded as an immortalized megakaryocyte cell line. These cells that were able to proliferate for 24 days or longer after infection were designated as immortalized megakaryocyte cell lines SeV2-MKCL and NIH5-MKCL. The obtained SeV2-MKCL and NIH5-MKCL were statically cultured in 10 cm dishes (10 mL/dish). The culture medium was IMDM as a basal medium supplemented with components given below (the concentration is a final concentration). Culture conditions were 27°C and 5% $CO_2$.

FBS (manufactured by Sigma-Aldrich Co. LLC, #172012, lot. 12E261) 15%
L-Glutamine (manufactured by Gibco/Thermo Fisher Scientific Inc., #25030-081) 2 mmol/L
ITS (manufactured by Gibco/Thermo Fisher Scientific Inc., #41400-045) 100-fold dilution
MTG (monothioglycerol, manufactured by Sigma-Aldrich Co. LLC, #M6145-25ML) 450 $\mu$mol/L
Ascorbic acid (manufactured by Sigma-Aldrich Co. LLC, #A4544) 50 $\mu$g/mL
Puromycin (manufactured by Sigma-Aldrich Co. LLC, #P8833-100MG) 2 $\mu$g/mL
SCF (manufactured by FUJIFILM Wako Pure Chemical Corp., #193-15513) 50 ng/mL
TPO-like agonist 200 ng/mL

(1-7) Production of culture product of megakaryocytes

Forced expression was canceled by culture in a DOX-free culture medium. Specifically, the immortalized megakaryocyte cell line (SeV2-MKCL and NIH5-MKCL) obtained by the method of the aforementioned (1-6) was washed twice with PBS(-) and suspended in a platelet production culture medium given below. The inoculation density of the cells was $1.0 \times 10^5$ cells/mL. Platelets were produced by culture for 6 days in the presence of the aforementioned platelet production culture medium to produce a culture product of megakaryocytes. The aforementioned platelet production culture medium was IMDM as a basal medium supplemented with components given below (the concentration is a final concentration).

Human plasma 5%

L-Glutamine (manufactured by Gibco/Thermo Fisher Scientific Inc., #25030-081) 4 mmol/L
ITS (manufactured by Gibco/Thermo Fisher Scientific Inc., #41400-045) 100-fold dilution
MTG (monothioglycerol, manufactured by Sigma-Aldrich Co. LLC, #M6145-25ML) 450 $\mu$mol/L
Ascorbic acid (manufactured by Sigma-Aldrich Co. LLC, #A4544) 50 $\mu$g/mL
SCF (manufactured by FUJIFILM Wako Pure Chemical Corp., #193-15513) 50 ng/mL
TPO-like agonist 200 ng/mL
ADAM inhibitor 15 $\mu$mol/L
GNF351 (manufactured by Calbiochem/Merck Millipore, #182707) 500 nmol/L
Y39983 (manufactured by Chemscene LLC, #CS-0096) 500 nmol/L
Urokinase 5 U/mL
Low-molecular heparin (manufactured by Sanofi S.A., Clexane) 1 U/mL

(1-8) Condensation of culture product of megakaryocytes

Platelets were manufactured (purified) from the culture product of megakaryocytes obtained in the aforementioned (1-7). The same purification was carried out twice. Specifically, the culture product of megakaryocytes obtained in the aforementioned (1-7) was introduced to a culture product bag. Then, the aforementioned culture product bag was connected to a condensation system, as shown in Figure 1. In Figure 1, washing and preserving-liquid bags 1 and 2 contain a washing and preserving-liquid. The aforementioned washing and preserving-liquid used was Bicanate infusion solution (manufactured by Otsuka Pharmaceutical Co., Ltd.) supplemented with 20% ACD and 2.5% human serum albumin and adjusted to pH 7.2 with NaOH. Then, the aforementioned culture product of megakaryocytes was condensed using a hollow fiber membrane (Plasma Flow OP, manufactured by Asahi Kasei Medical Co., Ltd.) according to Table 1 below, and the obtained condensed liquid of the culture product of megakaryocytes was recovered into a preservation bag.

[Table 1]

| Procedure | Relief valve | Pump 1 (rpm) | Pump 2 (rpm) | Contents |
|---|---|---|---|---|
| 1 | 2, 3, 4 | - | - | Introduce 50 mL of washing and preserving-liquid from washing and preserving-liquid bag 1 |
| 2 | 1, 3, 4 | - | - | Introduce 50 mL of washing and preserving-liquid from washing and preserving-liquid bag 1 |
| 3 | 5, 6 | - | 100 | Introduce 500 mL of washing and preserving-liquid from washing and preserving-liquid bag 2<br>Activate pump 2 for 1 min |
| 4 | 4, 7 | 50 | - | Introduce 500 mL of washing and preserving-liquid from washing and preserving-liquid bag 1<br>Activate pump 1 for 1 min |
| 5 | 1, 3, 6, 8 | 100 | 100 | Introduce culture product to hollow fiber |
| 6 | 2, 3, 6, 8 | 100 | 100 | Introduce condensation product to preservation |
| 7 | 2, 3, 4 | - | - | Introduce 50 mL of washing and preserving-liquid from washing and preserving-liquid bag 1 |
| 8 | 4, 8 | 50 | - | Introduce 500 mL of washing and preserving-liquid from washing and preserving-liquid bag 1 |

(1-9) Centrifugation of culture product

First, a waste bag of ACP215 disposable set was replaced with a bag for recovery using a sterile fusion apparatus. The aforementioned bag for recovery used was Hicaliq IVH bag (manufactured by Terumo Corp., HC-B3006A). Next, an ACD-A solution (manufactured by Terumo Corp.) was added in an amount of 10% to the aforementioned condensed liquid of the culture product of megakaryocytes. After the aforementioned addition, the condensed liquid supplemented with the ACD-A solution was injected to a cell bag. The aforementioned cell bag used was Hicaliq IVH bag (manufactured by Terumo Corp., HC-B3006A).

The cell bag containing the culture product supplemented with the ACD-A solution was fused to ACP215 disposable set using a sterile fusion apparatus. Then, ACP215 was started up on the service mode, and the number of revolutions was set to 2500 rpm (350 × g). ACP215 was started, and the culture product in the aforementioned cell bag was introduced at approximately 100 mL/min to a separation bowl. Liquid components effused from the aforementioned separation bowl were recovered into a recovery bag. After introduction of the whole amount of the culture product in the aforementioned cell bag to the separation bowl, 500 mL of a washing and preserving-liquid was further introduced to the aforementioned separation bowl. After introduction of the aforementioned washing and preserving-liquid to the aforementioned separation bowl, centrifugation was stopped, and the recovery bag containing the recovered liquid (recovered liquid components including platelets) was separated off using a tube sealer.

The recovery bag containing the recovered liquid (containing platelets) was fused to fresh ACP215 disposable set using the aforementioned sterile fusion apparatus. ACP215 was started up on the normal mode. WPC was selected for program setting, and the ACP215 disposable set fused with the aforementioned recovery bag was loaded therein according to the instruction of the equipment. The recovery bag containing the recovered liquid was installed in a stand.

Next, the centrifugation rate of ACP215 was changed to 5000 rpm (1398.8 × g), and centrifugation was started. Automated injection was changed to manual injection at the start of introduction of the aforementioned recovered liquid to the aforementioned separation bowl. Specifically, the aforementioned recovered liquid was introduced at an introduction rate of approximately 100 mL/min to the aforementioned separation bowl. After addition of the whole amount of the aforementioned recovered liquid to the separation bowl, 500 mL of a washing and preserving-liquid was further added.

(1-10) Washing of culture product

As for washing, the culture product was washed with 2000 mL of the aforementioned washing and preserving-liquid according to the program of ACP215.

(1-11) Recovery of culture product

200 mL of the washed culture product (containing platelets) was recovered into a platelet formulation bag according to the program of ACP215.

(1-12) Separation of culture product

Platelets were separated from the aforementioned platelet formulation bag by a routine method using the aforementioned hollow fiber membrane and recovered into a bag for recovery.

Example 2

[Addition of vitamin C to platelet preservation solution]

(2-1) Preparation of platelet preservation solution

A human serum albumin formulation (HSA; manufactured by CSL Behring K.K.) and a blood preservation solution (ACD-A solution; manufactured by Terumo Corp.) (2.20 W/V% sodium citrate hydrate, 0.80 W/V% citric acid hydrate, and 2.20 W/V% grape sugar) were added to a bicarbonate Ringer's solution (Bicanate infusion solution; manufactured by Otsuka Pharmaceutical Factory) (5.84 g/L sodium chloride, 0.30 g/L potassium chloride, 0.22 g/L calcium chloride hydrate, 0.20 g/L magnesium chloride, 2.35 g/L sodium bicarbonate, and 0.20 g/L sodium citrate hydrate) to prepare a solution. In the present specification, this solution is also referred to as a "first generation preservation solution". A VC formulation (injection formulation containing additives; manufactured by Sawai Pharmaceutical Co., Ltd.) was added to the first generation preservation solution to prepare a solution. In the present specification, this solution is also referred to as a "VC-supplemented preservation solution". The final concentrations of the aforementioned additives in each preservation solution are shown in Table 2 below. All the preservation solutions were adjusted to pH 7.3 ± 0.1 with 1 M NaOH and incubated for 1 hour or longer (in the dark, room temperature, 5% $CO_2$) until use.

[Table 2]

| | (1) First generation preservation solution | (2) First generation preservation solution | (3) VC-supplemented preservation solution | (4) VC-supplemented preservation solution | (5) VC-supplemented preservation solution |
|---|---|---|---|---|---|
| HSA (w/v%) | 2.5% | 2.5% | 2.5% | 2.5% | 2.5% |
| ACD-A solution (v/v%) | 20% | 10% | 20% | 20% | 20% |
| VC formulation | - | - | 300 mg/L | 1000 mg/L | 3000 mg/L |

(2-2) Preparation of platelet sample using each preservation solution

The concentration of platelets contained in the culture product obtained in Example 1 was measured by FACS. A necessary amount of the culture product containing platelets was separated. An ACD-A solution (10 v/v%) and PEG1 (final concentration: 2 $\mu$M; manufactured by Cayman Chemical Company) were added thereto, and the mixture was centrifuged for 12 minutes (1200 $\times$ g, 22°C). After removal of the supernatant, each preservation solution prepared in the aforementioned (2-1) was added to pellets, which were mildly suspended into a homogeneous liquid suspension (platelet concentration: approximately 0.3 $\times$ 10$^9$ plts/mL). Each liquid suspension was inoculated to a 24-well plate, preserved under horizontal shaking for up to 5 days (in the dark, 22°C, 50 rpm), and then subjected to experiments of the following (2-3) and (2-4).

(2-3) Annexin V positive ratio of platelet sample

Annexin V is an index for the deterioration (activation) of platelets. In the case of a high positive ratio, platelets are evaluated as being deteriorated or being abnormal. Accordingly, the Annexin V positive ratio of the platelet sample obtained in the aforementioned (2-2) was measured and examined for the effect of suppressing the deterioration of platelets by each preservation solution.

Specifically, the platelet sample obtained in the aforementioned (2-2) was diluted 500-fold with Annexin Buffer (manufactured by Becton, Dickinson and Company) and dispensed to three centrifugal tubes (for negative control, positive control, and unstimulated samples, respectively). EDTA was added to the negative control sample, and ionomycin was added to the positive control sample. Then, all the samples were stained with an anti-CD41 antibody (manufactured by BioLegend, Inc.) and Annexin V (manufactured by Becton, Dickinson and Company) (in the dark, room temperature, 20 min). Annexin Buffer was added to the samples thus stained, which were then immediately measured by FACS. The Annexin V positive ratio of iPS platelets (CD41$^+$ fraction) in the negative control was defined as 1.0 $\pm$ 0.1%. The Annexin V positive ratio of the unstimulated sample was calculated.

The results are shown in the upper part of Figure 2 (all the results were obtained from the platelet samples after preservation for 5 days). The Annexin V positive ratio of the platelets (CD41$^+$ fraction) was 60.5 $\pm$ 0.3 to 64.4 $\pm$ 0.1% in the case of using the first generation preservation solution and by contrast, was 53.9 $\pm$ 2.1 to 56.7 $\pm$ 1.8% in the case of using the VC-supplemented preservation solution, demonstrating reduction by the addition of VC. These results demonstrated that the addition of 300 to 3000 mg/L VC improves the platelet deterioration suppressive action of a platelet preservation solution.

(2-4) P-Selectin positive ratio in absence of stimulation, and PAC-1/P-selectin positive ratio in presence of ATR stimulation of platelet sample

P-Selectin is an index for the deterioration (activation) of platelets. In the case of a high P-selectin positive ratio in the absence of stimulation, platelets are evaluated as being deteriorated or being abnormal. Accordingly, the P-selectin positive ratio of the platelet sample obtained in the aforementioned (2-2) was measured and examined for the effect of suppressing the deterioration of platelets by each preservation solution.

In order to determine the preserved state of platelet functions, the PAC-1 and P-selectin positive ratio of each platelet sample stimulated with platelet activators adenosine diphosphate (ADP) and thrombin receptor activating peptide-6 (TRAP-6) was examined (hereinafter, the combination of ADP and TRAP-6 is also referred to as "ATR") .

Specifically, the platelet sample obtained in the aforementioned (2-2) was diluted 500-fold with Tyrode HEPES Buffer (THB) and dispensed to three centrifugal tubes (for negative control, positive control, and unstimulated samples, respectively). A mixed solution of ADP (manufactured by Sigma-Aldrich Co. LLC, final concentration: 20 $\mu$M) and TRAP-6 (manufactured by Bachem Holding AG, final concentration: 30 $\mu$M) was added to the positive control sample. The positive control sample thus stimulated and the unstimulated sample were stained with an anti-CD41 antibody (manufactured by BioLegend, Inc.), an anti-P-selectin antibody (manufactured by BioLegend, Inc.), and an anti-PAC-1 antibody (manufactured by Becton, Dickinson and Company) (in the dark, room temperature, 30 min). The negative control sample was stained with an anti-CD41 antibody (manufactured by BioLegend, Inc.), an isotype control antibody of the anti-P-selectin antibody (manufactured by BioLegend, Inc.), and an isotype control antibody of the anti-PAC-1 antibody (manufactured by BioLegend, Inc.) (in the dark, room temperature, 30 min). The samples thus stained were fixed by the addition of 1% paraformaldehyde (in the dark, 4°C, 30 min or longer), and a P-selectin and/or PAC-1 positive ratio was measured by FACS within 24 hours thereafter. For analysis, gating was performed such that the P-selectin positive ratio and PAC-1 positive ratio of iPS platelets (CD41$^+$ fraction) in the negative control sample were 1.0 $\pm$ 0.1% or less.

The intermediate part of Figure 2 shows the P-selectin positive ratio in the absence of stimulation, and the lower part of Figure 2 shows the PAC-1/P-selectin positive ratio in the presence of ATR stimulation (all the results were obtained from the platelet samples after preservation for 5 days). The P-selectin positive ratio of the unstimulated platelets (CD41$^+$ fraction) was 33.2 $\pm$ 0.3 to 45.6 $\pm$ 1.2% in the case of using the first generation preservation solution and by contrast, was 13.0 $\pm$ 0.2 to 19.7 $\pm$ 1.1% in the case of using the VC-supplemented preservation solution, demonstrating reduction by the addition of VC. The reduction in P-selectin positive ratio tended to depend on the concentration of VC added. These results showed that the addition of 300 to 3000 mg/L VC improves the platelet deterioration suppressive action of a platelet preservation solution.

The PAC-1/P-selectin positive ratio of the platelets (CD41$^+$ fraction) stimulated with ATR was 17.6 to 20.8% in the case of using the first generation preservation solution and by contrast, was 23.9 to 26.9% in the case of using the VC-supplemented preservation solution, demonstrating elevation by the addition of VC. These results showed that the addition of 300 to 3000 mg/L VC improves the platelet function maintaining action of a platelet preservation solution.

Example 3

[Addition of VC and VB3 to platelet preservation solution]

(3-1) Preparation of platelet preservation solution

Nicotinic acid (nicotinic acid injection formulation; manufactured by TOA EIYO Ltd.) was added to the VC-supplemented preservation solution to prepare a solution. As mentioned above, in the specification of the present application, the term "VB3" means nicotinic acid and/or nicotinamide. Nicotinic acid was used as VB3 in Examples 3 to 5 and 8 to 13, and nicotinic acid or nicotinamide was used as VB3 in Example 6. In the present specification, the solution of the VC-supplemented preservation solution supplemented with VB3 (nicotinic acid or nicotinamide) is also referred to as a "VC/VB3-supplemented preservation solution" (in accordance with the claims) or a "second generation preservation solution". The final concentrations of the aforementioned additives in each preservation solution are as shown in Table 3 below. All the preservation solutions were adjusted to pH 7.3 $\pm$ 0.1 with 1 M NaOH and incubated for 1 hour or longer (in the dark, room temperature, 5% $CO_2$) until use.

[Table 3]

| | First generation preservation solution | VC-supplemented preservation solution | VC/VB3-supplemented preservation solution (second generation preservation solution) |
|---|---|---|---|
| HSA (w/v%) | 2.5% | 2.5% | 2.5% |
| ACD-A solution (v/v%) | 20% | 20% | 20% |
| VC | - | 1000 mg/L | 1000 mg/L |
| Nicotinic acid | - | - | 400 mg/L |

(3-2) Preparation of platelet sample using each preservation solution

Each preservation solution prepared in the aforementioned (3-1) was added to an iPS cell-derived platelet formulation prepared by the method described in Example 1, which was mildly suspended into a homogeneous liquid suspension (platelet concentration: approximately 0.3 $\times$ 10$^9$ plts/mL). Each liquid suspension was subjected to experiments of the following (3-3) to (3-5) either immediately or after being inoculated to a 24-well plate and preserved under horizontal shaking for up to 5 days (in the dark, 22°C, 50 rpm).

(3-3) Annexin V positive ratio of platelet sample

The Annexin V positive ratio of the platelet sample (after preservation for 5 days) obtained in the aforementioned (3-2) was measured and examined for the effect of suppressing the deterioration of platelets by each preservation solution, according to the method described in (2-3) of Example 2.

The results are shown in Figure 3. The Annexin V positive ratio of the platelets (CD41$^+$ fraction) was $61.4 \pm 1.9\%$ in the case of using the first generation preservation solution, was $46.3 \pm 0.7\%$ in the case of using the VC-supplemented preservation solution, and was $44.3 \pm 0.5\%$ in the case of using the VC/VB3-supplemented preservation solution. These results demonstrated that use of VC and VB3 in combination more strongly suppresses the deterioration of platelets as compared with VC alone.

(3-4) Lactic acid production of platelet sample

It is known that lactic acid is produced by anaerobic metabolism in a platelet formulation during preservation and its pH is decreased with elevation in lactic acid concentration, resulting in the deterioration of platelets. Accordingly, the lactic acid concentration of the platelet sample obtained in the aforementioned (3-2) was measured and examined for the action of suppressing lactic acid production by each preservation solution.

Specifically, the platelet sample (after preservation for 5 days) obtained in the aforementioned (3-2) was placed in a 1.5 mL tube and centrifuged (1200 × g, 22°C, 10 min). The supernatant was recovered into a fresh tube and freeze-preserved at -80°C until measurement. The lactic acid concentration of the aforementioned supernatant was measured using N-Assay L LAC Nittobo (manufactured by Nittobo Medical Co., Ltd.) and automated analysis apparatus 7180 (manufactured by Hitachi High-Tech Corp.).

The results are shown in Figure 4. The lactic acid concentration of the sample supernatant was $0.35 \pm 0.01$ g/L in the case of using the first generation preservation solution, was $0.16 \pm 0.01$ g/L in the case of using the VC-supplemented preservation solution, and was $0.13 \pm 0.01$ g/L in the case of using the VC/VB3-supplemented preservation solution. These results demonstrated that use of VC and VB3 in combination more strongly suppresses the anaerobic metabolism of platelets during preservation as compared with VC alone.

(3-5) Platelet recovery ratio of platelet sample

The activation of platelets during preservation is considered to cause the formulation of aggregates and become responsible for decrease in platelet recovery ratio. Accordingly, the platelet concentration of the platelet sample before and after preservation obtained in the aforementioned (3-2) was measured and examined for change in the recovery ratio ascribable to each preservation solution.

Specifically, the platelet sample (before preservation and after preservation for 5 days) prepared in the aforementioned (3-2) was diluted 500-fold with THB and dispensed to TruCOUNT tubes (manufactured by Becton, Dickinson and Company). The sample was stained with an anti-CD41 antibody (manufactured by BioLegend, Inc.) and an anti-CD42b antibody (manufactured by BioLegend, Inc.) (in the dark, room temperature, 20 min), and THB was added thereto again, followed by measurement by FACS. The concentration of CD41$^+$ cells (platelets) was calculated on the basis of the bead count value of the TruCOUNT tube. A recovery ratio was calculated as to each platelet sample by using the platelet concentration before preservation as a denominator and the platelet concentration after preservation as a numerator.

The results are shown in Figure 5. The recovery ratio of the platelets was $102.0 \pm 4.2\%$ in the case of using the first generation preservation solution, was $101.1 \pm 6.8\%$ in the case of using the VC-supplemented preservation solution, and was $99.9 \pm 3.9\%$ in the case of using the VC/VB3-supplemented preservation solution. These results demonstrated that a recovery ratio does not differ by the addition of VC and/or VB3.

Example 4

[Addition of VC and nicotinic acid to platelet preservation solution]

(4-1) Preparation of platelet preservation solution and Preparation of platelet sample

A VC formulation (1000 mg/L, manufactured by Sawai Pharmaceutical Co., Ltd.) was added to the first generation preservation solution (containing 20% ACD-A solution) to prepare a VC-supplemented preservation solution. Nicotinic acid (400 mg/L, manufactured by TOA EIYO Ltd.) was added to the VC-supplemented preservation solution to prepare a second generation preservation solution (in accordance with the claims). The pH of each solution was adjusted to 7.3 ± 0.1. The makeup of the second generation preservation solution used in this Example is shown in Table 4 below.

[Table 4]

|  | MW | Second generation preservation solution (mg/L) |
|---|---|---|
| NaCl | 58.0 | 3953.68 |
| KCl | 75.0 | 203.10 |
| $CaCl_2 \cdot 2H_2O$ | 147.0 | 148.94 |
| $MgCl_2$ | 95.2 | 135.40 |
| Trisodium citrate dihydrate | 294.1 | 4535.40 |
| Citric acid monohydrate | 210.1 | 1600.00 |
| $NaHCO_3$ | 84.0 | 1590.95 |
| NaOH | 40.0 | 439.97 |
| Glucose | 180.0 | 4400.00 |
| Nicotinic acid (VB3) | 123.1 | 400.00 |
| Vitamin C | 176.1 | 1000.00 |
| L-Cysteine hydrochloride monohydrate | 175.6 | 8.00 |
| Sodium pyrosulfite | 190.1 | 8.00 |
| Benzyl alcohol | 108.1 | 120.00 |
| Albumin |  | 25000 |

The aforementioned preservation solution (first generation preservation solution, VC-supplemented preservation solution, or second generation preservation solution) was added to an iPS cell-derived platelet formulation prepared by the method described in Example 1, which was mildly suspended into a homogeneous liquid suspension (platelet concentration: approximately $1.0 \times 10^9$ plts/mL). Each liquid suspension was subjected to experiments of the following (4-2) to (4-4) either immediately or after being packed into a blood preservation bag and preserved under horizontal shaking for 5 or 10 days (in the dark, 22°C, 50 rpm).

(4-2) Lactic acid concentration and pH of platelet sample

As also mentioned in the aforementioned (2-4), it is known that lactic acid is produced by anaerobic metabolism in a platelet formulation during preservation and becomes responsible for decrease in pH. In this experiment, the lactic acid concentration and pH of the platelet sample obtained in the aforementioned (4-1) were measured and examined for the action of suppressing lactic acid production by each preservation solution.

Specifically, the platelet sample (before preservation, after preservation for 5 days, and after preservation for 10 days) obtained in the aforementioned (4-1) was placed in a 1.5 mL tube, and a lactic acid concentration and pH were measured with analysis apparatus FLEX for cell culture (manufactured by Nova Biomedical Corp.).

The results are shown in Figure 6. As shown in the left graph of Figure 6, the lactic acid concentration was elevated over time in the case of using any of the preservation solutions, whereas the degree of elevation was lowest in the sample containing the second generation preservation solution. As shown in the right graph of Figure 6, the pH was decreased over time in the case of using any of the preservation solutions, whereas the degree of decrease was lowest in the sample containing the second generation preservation solution and was kept neutral even after preservation for 10 days.

(4-3) P-Selectin positive ratio in absence of stimulation, and PAC-1/P-selectin positive ratio in presence of ATR stimulation of platelet sample

As also mentioned in (2-4) of Example 2, P-selectin is known as a deterioration marker for platelets, and PAC-1/P-selectin in the presence of ATR (ADP/TRAP-6) stimulation is known as a reactivity marker for platelets. Accordingly, in this experiment, the P-selectin positive ratio in the absence of stimulation, and PAC-1/P-selectin positive ratio in the presence of ATR stimulation of the platelet sample obtained in the aforementioned (4-1) were measured and examined for the effect of suppressing the deterioration of platelets by each preservation solution, according to the method described in (2-4) of Example 2.

The P-selectin positive ratio in the absence of stimulation is shown on the left in Figure 7, and the PAC-1/P-selectin positive ratio in the presence of ATR stimulation is shown on the right in Figure 7. The P-selectin positive ratio was 18.0% before preservation (Day 1), which was however elevated to 33.0% after preservation for 5 days (Day 5) and 31.1% after preservation for 10 days (Day 10), in the case of using the first generation preservation solution. On the other hand, the P-selectin positive ratio after preservation for 5 and 10 days in the case of using the VC-supplemented preservation solution was low as compared with the case of using the first generation preservation solution and was 26.2% (Day 5) and 24.4% (Day 10), respectively. The P-selectin positive ratio after preservation for 5 and 10 days in the case of using the second generation preservation solution was much lower than that in the case of using the VC-supplemented preservation solution and was 24.2% (Day 5) and 19.3% (Day 10), respectively. These results demonstrated that use of the second generation preservation solution suppresses the deterioration of platelets even after preservation for 10 days.

The PAC-1/P-selectin positive ratio in the presence of ATR stimulation was 43.4% before preservation (Day 1), which was however rapidly decreased to 34.5% after preservation for 5 days (Day 5) and 5.9% after preservation for 10 days (Day 10), in the case of using the first generation preservation solution. On the other hand, the PAC-1/P-selectin positive ratio in the presence of ATR stimulation in the case of using the VC-supplemented preservation solution or the second generation preservation solution was at the same level (35.9% and 35.3%, respectively) as in the case of using the first generation preservation solution after preservation for 5 days (Day 5) and exhibited a much higher value (19.4% and 19.9%, respectively) than that for the first generation preservation solution after preservation for 10 days (Day 10). These results demonstrated that use of the VC-supplemented preservation solution or the second generation preservation solution maintains the reactivity of platelets even after preservation for 10 days.

(4-4) Platelet recovery ratio of platelet sample

The platelet concentration and recovery ratio of the platelet sample obtained in the aforementioned (4-1) were measured by the method described in the aforementioned (3-5).

The results are shown in Figure 8. The platelet recovery ratio in the case of using the first generation preservation solution was 85.4% after preservation for 5 days (Day 5) and 86.8% after preservation for 10 days (Day 10). On the other hand, the platelet recovery ratio in the case of using the VC-supplemented preservation solution or the second generation preservation solution was 90% or more (90.0% and 91.4%, respectively) even after preservation for 10 days (Day 10). These results are consistent with the results of the aforementioned (4-3) showing that use of the VC-supplemented preservation solution or the second generation preservation solution suppresses the activation of platelets during preservation.

Example 5

[Aggregation capacity of platelet sample]

(5-1) Preparation of preservation solution and platelet sample

The first generation preservation solution and the second generation preservation solution were prepared by the method described in (3-1) of Example 3. The aforementioned preservation solution was added to an iPS cell-derived platelet formulation prepared by the method described in Example 1, which was mildly suspended into a homogeneous liquid suspension (platelet concentration: approximately $1.0 \times 10^9$ plts/mL). Each liquid suspension was subjected to an experiment of the following (5-2) either immediately or after being packed into a blood preservation bag and preserved under horizontal shaking for 5 or 10 days (in the dark, 22°C, 50 rpm).

(5-2) Aggregation capacity measurement of platelet sample

The platelet sample obtained in the aforementioned (5-1) was centrifuged (1200 $\times$ g, room temperature, 10 min) to remove a supernatant. The sample was suspended by the addition of a bicarbonate Ringer's solution containing 5% ACD-A such that the cell concentration was $1.0 \times 10^9$ plts/ml. The obtained liquid suspension was diluted with human plasma/$CaCl_2$ solution (manufactured by Cosmo Bio Co., Ltd.) and simulated by the addition of the stimulant shown in Table 5 below. The aggregation ratio of each sample thus stimulated was measured with platelet aggregation capacity measurement apparatus PRP313M (manufactured by TAIYO Instruments, Inc.).

[Table 5]

| Stimulant name | Final concentration |
|---|---|
| TRAP-6 | 10 $\mu$M |
| | 20 $\mu$M |
| | 40 $\mu$M |
| Collagen | 2.5 $\mu$g/mL |
| | 5 $\mu$g/mL |
| | 10 $\mu$g/mL |
| ADP | 5 $\mu$M |
| | 10 $\mu$M |
| | 20 $\mu$M |
| Collagen/ADP | 2.5 $\mu$g/mL, 5 $\mu$M |
| | 5 $\mu$g/mL, 5 $\mu$M |

Figures 9 to 12 show results of using TRAP-6, collagen, ADP, and collagen/ADP, respectively, as a stimulant. It was revealed that the maximum aggregation ratio of the platelet sample containing the first generation preservation solution was drastically decreased during preservation for 5 to 10 days in the case of using any of the stimulants ("Conventional preservation solution (Day 10)" in Figures 9 to 12). On the other hand, such rapid decrease in aggregation ratio was not found in the platelet sample containing the second generation preservation solution ("Novel preservation solution (Day 10)" in Figures 9 to 12). These results demonstrated that use of the second generation preservation solution can maintain the aggregation capacity (hemostatic capacity) of platelets during preservation for 10 days or longer.

Example 6

[Addition of nicotinic acid or nicotinamide to preservation solution]

(6-1) Preparation of preservation solution and platelet sample

VC (1000 mg/L) and nicotinic acid (400 mg/L; manufactured by FUJIFILM Wako Pure Chemical Corp.) or nicotinamide (400 mg/L; manufactured by FUJIFILM Wako Pure Chemical Corp.) were added to the first generation preservation solution (containing 20% ACD-A solution), and the mixture was adjusted to pH 7.3 $\pm$ 0.1 using 1 M NaOH. Hereinafter, the preservation solution containing the first generation preservation solution supplemented with VC and nicotinic acid is also referred to as a "second generation preservation solution (nicotinic acid)" (in accordance with the claims), and the preservation solution containing the first generation preservation solution supplemented with VC and nicotinamide is also referred to as a "second generation preservation solution (nicotinamide)".
The aforementioned preservation solution was added to an iPS cell-derived platelet formulation prepared by the method described in Example 1, which was mildly suspended into a homogeneous liquid suspension (platelet concentration: approximately $0.3 \times 10^9$ plts/mL). Each liquid suspension was subjected to experiments of the following (6-2) and (6-3) either immediately or after being inoculated to a 24-well plate and preserved under horizontal shaking for 5 days (in the dark, 22°C, 50 rpm).

(6-2) Change in Annexin V positive ratio of platelet sample

The Annexin V (deterioration marker) positive ratio of the platelet sample obtained in the aforementioned (6-1) was measured and examined for the effect of suppressing the deterioration of platelets by each preservation solution, according to the method described in (2-3) of Example 2.
The results are shown in the upper part of Figure 13. In the diagram, "Day 1" depicts the sample before preservation, and "First generation", "Second generation (nicotinic acid)", and "Second generation (nicotinamide)" each depict the sample after preservation for 5 days. The Annexin V positive ratio of the platelets (CD41$^+$ fraction) was 38.2 $\pm$ 0.3% for the preservation solution supplemented with nicotinic acid, and was 38.5 $\pm$ 0.3% for the preservation solution supplemented with nicotinamide. Both of these values were lower than that in the case of using the first generation preservation solution (53.1 $\pm$ 0.4%). These results demonstrated that even vitamin B3 in the broad sense (nicotinic acid and/or nicotinamide) suppresses the deterioration of platelets during preservation.

(6-3) Influence of each preservation solution on P-selectin positive ratio in absence of stimulation, and PAC-1/P-selectin positive ratio in presence of ATR stimulation of platelet sample

The P-selectin (deterioration marker) positive ratio in the absence of stimulation, and PAC-1/P-selectin (reactivity marker) positive ratio in the presence of ATR stimulation of the platelet sample obtained in the aforementioned (6-1) were measured and examined for the effect of suppressing the deterioration of platelets by each preservation solution, according to the method described in (2-4) of Example **2.**

The P-selectin positive ratio in the absence of stimulation is shown in the intermediate part of Figure 13, and the PAC-1/P-selectin positive ratio in the presence of ATR stimulation is shown in the lower part of Figure 13. The P-selectin positive ratio was 11.6% before preservation (Day 1) which was elevated to $28.3 \pm 0.5\%$ after preservation for 5 days (Day 5), in the case of using the first generation preservation solution. On the other hand, the P-selectin positive ratio in the case of using the second generation preservation solution supplemented with nicotinic acid or nicotinamide was kept as low as $9.4 \pm 0.2\%$ to $10.6 \pm 0.2\%$ even after preservation for 5 days.

The PAC-1/P-selectin positive ratio in the presence of ATR stimulation was $33.9 \pm 2.4\%$ in the case of using the first generation preservation solution, and was $41.6 \pm 0.8\%$ to $41.2 \pm 1.9\%$ in the case of using the second generation preservation solution supplemented with nicotinic acid or nicotinamide. These results demonstrated that nicotinic acid or nicotinamide maintains the reactivity of platelets during preservation.

Example 7

[Addition of VC alone to preservation solution]

(7-1) Preparation of preservation solution and platelet sample

In the aforementioned Examples 2 to 6, the VC formulation manufactured by Sawai Pharmaceutical Co., Ltd. was used as "VC". Since the VC formulation contains additives consisting of sodium pyrosulfite, L-cysteine hydrochloride monohydrate and benzyl alcohol, whether these additives influenced experimental results was confirmed by the following experiment.

A VC reagent (additive-free VC; 1000 mg/L, manufactured by FUJIFILM Wako Pure Chemical Corp.) was added to the first generation preservation solution (containing 20% ACD-A solution), and the mixture was adjusted to pH $7.3 \pm 0.1$. Hereinafter, this preservation solution is also referred to as a "first generation preservation solution (VC reagent)". The aforementioned preservation solution was added to an iPS cell-derived platelet formulation prepared by the method described in Example 1, which was mildly suspended into a homogeneous liquid suspension (platelet concentration: approximately $0.3 \times 10^9$ plts/mL). Each liquid suspension was subjected to experiments of the following (7-2) and (7-3) either immediately or after being inoculated to a 24-well plate and preserved under horizontal shaking for 5 days (in the dark, 22°C, 50 rpm).

(7-2) Annexin V positive ratio of platelet sample

The Annexin V (deterioration marker) positive ratio of the platelet sample obtained in the aforementioned (7-1) was measured and examined for the effect of suppressing the deterioration of platelets by each preservation solution, according to the method described in (2-3) of Example 2.

The results are shown in the upper part of Figure 14 ("Day 1" depicts the sample before preservation, and "First generation" and "First generation VC reagent" each depict the sample after preservation for 5 days). The Annexin V positive ratio of the platelets (CD41$^+$ fraction) was lower in the case of using the first generation preservation solution (VC reagent) ($38.9 \pm 0.4\%$) than in the case of using the first generation preservation solution ($53.1 \pm 0.4\%$). These results demonstrated that the addition of VC alone (containing no VB3) suppresses the deterioration of iPS-derived platelets during preservation.

(7-3) P-Selectin positive ratio in absence of stimulation, and PAC-1/P-selectin positive ratio in presence of ATR stimulation of platelet sample

The P-selectin (deterioration marker) positive ratio in the absence of stimulation, and PAC-1/P-selectin (reactivity marker) positive ratio in the presence of ATR stimulation of the platelet sample obtained in the aforementioned (7-1) were measured and examined for the effect of suppressing the deterioration of platelets by each preservation solution, according to the method described in (2-4) of Example 2.

The P-selectin positive ratio in the absence of stimulation is shown in the intermediate part of Figure 14, and the PAC-1/P-selectin positive ratio in the presence of ATR stimulation is shown in the lower part of Figure 14. The P-selectin positive ratio in the absence of stimulation was lower in the case of using the first generation preservation solution (VC reagent) ($9.5 \pm 0.4\%$) than in the case of using the first generation preservation solution ($28.3 \pm 0.5\%$). These results demonstrated that the addition of VC alone suppresses the deterioration of iPS-derived platelets during preservation.

The PAC-1/P-selectin positive ratio in the presence of ATR stimulation was higher in the case of using the first generation preservation solution (VC reagent) ($38.2 \pm 1.6\%$) than in the case of using the first generation preservation solution ($33.9 \pm 2.4\%$). These results demonstrated that the addition of VC alone maintains the reactivity of iPS-derived platelets during preservation. Specifically, it was revealed that the effect brought about by the addition of VC was not due to the additives contained in the VC formulation manufactured by Sawai Pharmaceutical Co., Ltd.

Example 8

[Comparison with NISSEKI platelet washing-solution]

(8-1) Preparation of preservation solution and platelet sample

The additives shown in Table 6 below were added to a Bicanate infusion solution to prepare a second generation preservation solution. A solution having the same makeup as that of a known platelet preservation solution (Japanese Red Cross Society, the package insert of Irradiated Washed Platelets-LR "NISSEKI", March 2016, and Japanese Journal of Transfusion and Cell Therapy, Vol. 59. No. 3, 59 (3): 492-498, 2013) was prepared. In the present specification, this known preservation solution is also referred to as "NISSEKI platelet washing-solution". Both the preservation solutions were adjusted to pH $7.3 \pm 0.1$ with 1 M NaOH and incubated for 1 hour or longer (in the dark, room temperature, 5% $CO_2$) until use. The aforementioned preservation solution was added to an iPS cell-derived platelet formulation prepared by the method described in Example 1, which was mildly suspended into a homogeneous liquid suspension (platelet concentration: approximately $1.0 \times 10^9$ plts/mL). Each liquid suspension was subjected to experiments of the following (8-2) and (8-3) either immediately or after being packed into a blood preservation bag and preserved under horizontal shaking for 5 days (in the dark, 22°C, 50 rpm).

[Table 6]

| | Second generation preservation solution | NISSEKI platelet washing-solution |
|---|---|---|
| HSA (w/v%) | 2.5% | - |
| ACD-A solution (v/v%) | 20% | 5% |
| VC formulation (manufactured by Sawai Pharmaceutical Co., Ltd.) | 1000 mg/L | - |
| Nicotinic acid (manufactured by TOA EIYO Ltd.) | 400 mg/L | - |

(8-2) Annexin V positive ratio of platelet sample

The Annexin V (deterioration marker) positive ratio of the platelet sample obtained in the aforementioned (8-1) was measured and examined for the effect of suppressing the deterioration of platelets by each preservation solution, according to the method described in (2-3) of Example 2.

The results are shown in the upper part of Figure 15 ("Day 1" depicts the sample before preservation, and "Day 5" depicts the sample after preservation for 5 days). The Annexin V positive ratio of the platelets (CD41+ fraction) was lower in the case of using the second generation preservation solution (49.3%) than in the case of using the NISSEKI platelet washing-solution (73.9%). These results demonstrated that the second generation preservation solution suppresses the deterioration of iPS-derived platelets during preservation.

(8-3) P-Selectin positive ratio in absence of stimulation, and PAC-1/P-selectin positive ratio in presence of ATR stimulation of platelet sample

The P-selectin (deterioration marker) positive ratio in the absence of stimulation, and PAC-1/P-selectin (reactivity marker) positive ratio in the presence of ATR stimulation of the platelet sample obtained in the aforementioned (8-1) were measured and examined for the effect of suppressing the deterioration of platelets by each preservation solution, according to the method described in (2-4) of Example 2.

The P-selectin positive ratio in the absence of stimulation is shown in the intermediate part of Figure 15, and the PAC-1/P-selectin positive ratio in the presence of ATR stimulation is shown in the lower part of Figure 15. The P-selectin positive ratio in the absence of stimulation was lower in the case of using the second generation preservation solution (16.5%) than in the case of using the NISSEKI platelet washing-solution (30.4%). These results demonstrated that the second generation preservation solution exerts a better deterioration suppressive effect than that of the NISSEKI platelet washing-solution on iPS-derived platelets during preservation.

The PAC-1/P-selectin positive ratio in the presence of ATR stimulation was higher in the case of using the second generation preservation solution (24.8%) than in the case of using the NISSEKI platelet washing-solution (9.8%). These results demonstrated that the second generation preservation solution exerts a better function maintaining effect than that of the NISSEKI platelet washing-solution on iPS-derived platelets during preservation.

Example 9

[Hemostasis test using thrombocytopenic model mouse]

(9-1) Preparation of platelet sample

The second generation preservation solution was added to an iPS cell-derived platelet formulation prepared by the method described in Example 1, which was mildly suspended into a homogeneous liquid suspension (platelet concentration: approximately $1.0 \times 10^9$ plts/mL). Each liquid suspension was subjected to an experiment of the following (9-2) either immediately or after being packed into a blood preservation bag and preserved under horizontal shaking (in the dark, 22°C, 50 rpm, 10 days).

(9-2) Hemostasis test

The platelet sample obtained in the aforementioned (9-1) was administered into the tail vein of each thrombocytopenic model NOG mouse (200 μL ($2 \times 10^8$ plts) per mouse). 10 minutes after administration, an incision wound was made in the ventral tail artery using an injection needle. The incision wound was made at one location per individual. After confirmation of bleeding from the incision wound, the tail tip including the incision wound location was dipped in saline of 37°C, and the time to hemostasis was measured. The measurement time was 600 seconds at the maximum. A similar test was conducted using only the second generation preservation solution (free from platelets) as a control group (vehicle).

The results are shown in Figure 16. Hemostasis within 600 seconds was not found in all the mice in the vehicle administration group. On the other hand, the time to hemostasis in the platelet administration group was 391 seconds on average and was 130 seconds in an individual with the shortest time. These results demonstrated that iPS cell-derived platelets preserved in the second generation preservation solution of the present invention have a hemostatic effect.

Example 10

[Preservation of human mesenchymal stem cells]

(10-1) Preparation of mesenchymal stem cell preservation solution

3% trehalose and 5% dextran were added to a lactated Ringer's solution (Lactec infusion solution; manufactured by Otsuka Pharmaceutical Factory) (hereinafter, this solution is also referred to as a "CSP-01 solution"; see Japanese unexamined Patent Application Publication No. 2012-115253 and WO2014/208053). A VC formulation (1000 mg/L, manufactured by Sawai Pharmaceutical Co., Ltd.) and nicotinic acid (400 mg/L, manufactured by TOA EIYO Ltd.) were added to the aforementioned CSP-01 solution to prepare a mesenchymal stem cell preservation solution (in accordance with the claims). Distilled water (Otsuka Distilled Water; manufactured by Otsuka Pharmaceutical Factory) was added instead of VC and nicotinic acid to the CSP-01 solution to prepare a control preservation solution.

(10-2) Preservation of mesenchymal stem cells

Human bone marrow-derived mesenchymal stem cells (manufactured by Lonza Group AG) were suspended (5 $\times 10^5$ cells/mL) using each preservation solution prepared in the aforementioned (10-1). The liquid suspension was left standing at 5°C for 24, 48, 96, and 168 hours. Then, the total cell number and a dead cell number were counted under a microscope. Cell viability (%) and a viable cell recovery ratio (%) at each point in time were calculated according to the following equations 1 and 2.

Cell viability (%) = (Total cell number - Dead cell number) / Total cell number $\times$ 100 [Equation 1]

Viable cell recovery ratio (%) = Viable cell number at each point in time / Viable cell number immediately after suspension (before preservation) $\times$ 100 [Equation 2]

The results are shown in Figure 17. It was revealed that the cell viability and the viable cell recovery ratio in the case of using the control preservation solution were rapidly decreased after preservation for 48 hours. On the other hand, no drastic decrease in cell viability and viable cell recovery ratio was found in the case of using the preservation solution supplemented with VC and nicotinic acid, demonstrating that the same level as that before preservation was maintained even after 168 hours. These results showed that VC and nicotinic acid also exert an excellent effect on the preservation of mesenchymal stem cells.

Example 11

[Preservation of iPS cell-derived megakaryocytes]

(11-1) Preparation of iPS cell-derived megakaryocytes

The concentration of platelets contained in the culture product obtained in Example 1 was measured by FACS. A necessary amount of the culture product was separated. An ACD-A solution (10 v/v%) and PEG1 (final concentration: 2 $\mu$M; manufactured by Cayman Chemical Company) were added thereto, and the mixture was centrifuged for 12 minutes (1200 $\times$ g, 22°C, minimum brake). After removal of the supernatant, the first generation or second generation preservation solution was added to pellets, which were mildly suspended into a homogeneous liquid suspension (platelet concentration: approximately $1.3 \times 10^9$ plts/mL). Each liquid suspension was subjected to an experiment of the following (11-2) either immediately or after being packed into a blood preservation bag and preserved under horizontal shaking for 5 or 10 days (in the dark, 22°C, 50 rpm).

(11-2) Annexin V negative ratio of megakaryocyte sample

Annexin V is also known as a probe for detecting change in cell membrane (appearance of phosphatidylserine outside cell membranes) in apoptotic cells. Accordingly, the Annexin V negative ratio of the megakaryocyte sample obtained in the aforementioned (11-1) was measured and examined for the effect of suppressing the apoptosis of megakaryocytes by each preservation solution.

Specifically, the megakaryocyte sample (culture product containing megakaryocytes and platelets) obtained in the aforementioned (11-1) was diluted 500-fold with Annexin Buffer (manufactured by Becton, Dickinson and Company) and dispensed to three centrifugal tubes (for negative control, positive control, and unstimulated samples, respectively). EDTA was added to the negative control sample, and ionomycin was added to the positive control sample. Then, all the samples were stained with an anti-CD41 antibody (manufactured by BioLegend, Inc.) and Annexin V (manufactured by Becton, Dickinson and Company) (in the dark, room temperature, 20 min). Annexin Buffer was added to the samples thus stained, which were then immediately measured by FACS. Platelets and megakaryocytes were sorted out on the basis of the obtained values of FSC (forward scatter) and SSC (side scatter). The Annexin V positive ratio of iPS platelets (CD41[+] fraction) in the negative control was defined as $1.0 \pm 0.1\%$. The Annexin V negative ratio of megakaryocytes in the unstimulated sample was calculated.

The results are shown in Figure 18 ("Day 1" depicts the sample before preservation, "Day 5" depicts the sample after preservation for 5 days, and "Day 10" depicts the sample after preservation for 10 days). The Annexin V negative ratio was decreased over time both in the sample containing the first generation preservation solution and in the sample containing the second generation preservation solution. However, it was revealed that decrease in Annexin V negative ratio is suppressed in the case of using the second generation preservation solution as compared with the case of using the first generation preservation solution. These results showed that non-apoptotic viable cells having a stable cell membrane are contained at a high proportion in a sample containing the second generation preservation solution. Thus, this Example showed that the second generation preservation solution is also effective for preserving iPS cell-derived megakaryocytes.

Example 12

[Preservation of T cells]

(12-1) Preparation of T cell preservation solution

3% trehalose was added to a lactated Ringer's solution (Lactec infusion solution; manufactured by Otsuka Pharmaceutical Factory) (hereinafter, this solution is referred to as a "CSP-11 solution"). A VC formulation (1000 mg/L, manufactured by Sawai Pharmaceutical Co., Ltd.) and/or nicotinic acid (400 mg/L, manufactured by TOA EIYO Ltd.) was added to the aforementioned CSP-11 solution to prepare the following four types of T cell preservation solutions.

CSP-11
CSP-11 + VC
CSP-11 + nicotinic acid
CSP-11 + VC + nicotinic acid (in accordance with the claims)

(12-2) Preservation of T cells

Commercially available frozen CD8-positive T cells (manufactured by VERITAS Corp.) were thawed, washed with a lymphocyte culture medium (LGM3, manufactured by Lonza Group AG), and then incubated for approximately 1 hour (37°C, 5% $CO_2$). A necessary amount of the CD8-positive T cells was separated and centrifuged for 10 minutes (300 × g, room temperature). After removal of the supernatant, the cells were floated using TLY CULTURE Kit 25 (manufactured by GC Lymphotec Inc.) and cultured under conditions of 37°C and 5% $CO_2$ for proliferation (T cell concentration: approximately 1.1 × $10^6$ cells/5 ml). 7 days after the start of expansion culture, the cells were washed with a CSP-11 solution, dispensed to STEMFULL tubes (manufactured by Sumitomo Bakelite Co., Ltd.), and centrifuged for 10 minutes (300 × g, room temperature). After removal of the supernatant, the cells were suspended by the addition of each preservation solution prepared in the aforementioned (12-1) (T cell concentration: approximately 5 × $10^5$ cells/1 ml). 20 μL of the liquid suspension of the cells was separated from each STEMFULL tube and mixed with 20 μL of Trypan Blue (manufactured by Gibco/Thermo Fisher Scientific Inc.), and viability was measured using OneCell Counter (manufactured by Bio Medical Sciences Co., Ltd.) (the total cell number and a dead cell number of areas of cell counting rooms at four corners of one cell counting part were counted). Also, the aforementioned liquid suspension of the cells was preserved at 5°C for 48 hours, and viability was measured in the same way as above.

The results are shown in Figure 19. The viability before preservation did not differ among the preservation solutions (Figure 19). On the other hand, it was revealed that the viability after preservation for 48 hours was significantly higher by use of VC alone, or the preservation solution supplemented with VC and nicotinic acid (Figure 19). Furthermore, significantly higher viability was exhibited by use of the preservation solution supplemented with VC and nicotinic acid as compared with VC alone. Thus, the preservation solution of the present invention was shown to be also effective for refrigerating T cells.

Example 13

[Preservation of T cells]

(13-1) Preparation of T cell preservation solution

A VC formulation (1000 mg/L, manufactured by Sawai Pharmaceutical Co., Ltd.), nicotinic acid (400 mg/L, manufactured by TOA EIYO Ltd.) and/or glucose (80 mg/dL, manufactured by Otsuka Pharmaceutical Factory) was added to the CSP-01 solution to prepare the following four types of T cell preservation solutions.

CSP-01
CSP-01 + VC + nicotinic acid (in accordance with the claims)
CSP-01 + glucose
CSP-01 + glucose + VC + nicotinic acid (in accordance with the claims)

(13-2) Preservation of T cells

Commercially available frozen CD8-positive T cells (manufactured by VERITAS Corp.) were thawed, washed with a lymphocyte culture medium (LGM3, manufactured by Lonza Group AG), and then incubated for approximately 6 hours (37°C, 5% $CO_2$). A necessary amount of the CD8-positive T cells was separated and centrifuged for 10 minutes (300 × g, room temperature). After removal of the supernatant, the cells were floated using TLY CULTURE Kit 25 (manufactured by GC Lymphotec Inc.) and cultured under conditions of 37°C and 5% $CO_2$ for proliferation (T cell concentration: approximately 1.2 × $10^6$ cells/5 ml). 6 days after the start of expansion culture, the cells were washed with a lactated Ringer's solution containing 3% trehalose, dispensed to STEMFULL tubes (manufactured by Sumitomo Bakelite Co., Ltd.), and centrifuged for 10 minutes (300 $\times$ g, room temperature). After removal of the supernatant, the cells were suspended by the addition of each preservation solution prepared in the aforementioned (12-1) (T cell concentration: approximately $5 \times 10^5$ cells/1 ml). 20 $\mu$L of the liquid suspension of the cells was separated from each STEMFULL tube and mixed with 20 $\mu$L of Trypan Blue (manufactured by Gibco/Thermo Fisher Scientific Inc.), and viability was measured using OneCell Counter (manufactured by Bio Medical Sciences Co., Ltd.) (the total cell number and a dead cell number of areas of cell counting rooms at four corners of one cell counting part were counted). Also, the aforementioned liquid suspension of the cells was preserved at 5°C for 24 or 48 hours, and viability was measured in the same way as above. A viable cell recovery ratio was calculated according to the following equation 3 from the obtained viability at each point in time.

Viable cell recovery ratio (%) = (Viable cell number after preservation) / (Viable cell number before preservation) $\times$ 100 [Equation 3]

The results are shown in Figure 20. The viability before preservation did not differ among the preservation solutions (left graph of Figure 20). On the other hand, it was revealed that the viability and the viable cell recovery ratio after preservation for 24 and 48 hours were higher by use of the preservation solution supplemented with VC and nicotinic acid (central and right graphs of Figure 20). Thus, the preservation solution of the present invention was shown to be also effective for refrigerating T cells. It was also revealed that the addition of glucose in addition to VC and nicotinic acid tends to improve viability and a viable cell recovery ratio.

Example 14

[Inhibitory action of VB2]

(14-1) Preparation of preservation solution supplemented with water-soluble vitamin group

A water-soluble vitamin group (B1, VB2, VB3, VB5, VB6, VB7, VB9, VB12, and VC) was added to the first generation preservation solution (containing 20% ACD-A solution) (hereinafter, this preservation solution is also referred to as "first generation + water-soluble vitamin"). Also, a group of the aforementioned water-soluble vitamin group except for VB2 (B1, VB3, VB5, VB6, VB7, VB9, VB12, and VC) was added to the first generation preservation solution (containing 20% ACD-A solution) (hereinafter, this preservation solution is also referred to as "first generation + water-soluble vitamin (except for VB2)" (in accordance with the claims)). Both the preservation solutions were adjusted to pH 7.3 $\pm$ 0.1 using 1 M NaOH.

The aforementioned preservation solution was added to an iPS cell-derived platelet formulation prepared by the method described in Example 1, which was mildly suspended into a homogeneous liquid suspension (platelet concentration: approximately $0.3 \times 10^9$ plts/mL). Each liquid suspension was subjected to experiments of the following (14-2) and (14-3) either immediately or after being inoculated to a 24-well plate and preserved under horizontal shaking for 5 days (in the dark, 22°C, 50 rpm).

(14-2) Change in Annexin V positive ratio of platelet sample

The Annexin V (deterioration marker) positive ratio of the platelet sample obtained in the aforementioned (14-1) was measured and examined for the effect of suppressing the deterioration of platelets by each preservation solution, according to the method described in (2-3) of Example **2**.

The results are shown in Figure 21. In the diagram, "Day 1" depicts the sample before preservation, and "Day **5**" depicts the sample after preservation for 5 days. The Annexin V positive ratio of the platelets (CD41$^+$ fraction) after preservation for 5 days was 52.0% in the case of using the first generation preservation solution, which was however decreased to 43.0% for the preservation solution supplemented with the water-soluble vitamin group. The Annexin V positive ratio was further decreased to 40.3% for the preservation solution supplemented with the water-soluble vitamin group (except for VB2). These results showed the possibility that the effect of suppressing platelet deterioration by VC and VB3 is inhibited by VB2.

(14-3) Influence of each preservation solution on P-selectin positive ratio in absence of stimulation, and PAC-1/P-selectin positive ratio in presence of ATR stimulation of platelet sample

The P-selectin (deterioration marker) positive ratio in the absence of stimulation, and PAC-1/P-selectin (reactivity marker) positive ratio in the presence of ATR stimulation of the platelet sample obtained in the aforementioned (14-1) were measured and examined for the effect of suppressing the deterioration of platelets by each preservation solution, according to the method described in (2-4) of Example **2**.

The P-selectin positive ratio in the absence of stimulation is shown on the left side in Figure 22, and the PAC-1/P-selectin positive ratio in the presence of ATR stimulation is shown on the right side in Figure 22. The P-selectin positive ratio was 27.4% before preservation (Day 1) which was elevated to 42.9% after preservation for 5 days (Day 5), in the case of using the first generation preservation solution. On the other hand, the P-selectin positive ratio after preservation for 5 days was kept as low as 34.9% in the case of using the preservation solution supplemented with the water-soluble vitamin group, and kept lower (24.0%) in the case of using the preservation solution supplemented with the water-soluble vitamin group (except for VB2).

The PAC-1/P-selectin positive ratio in the presence of ATR stimulation was 35.6% before preservation (Day 1) which was decreased to 32.4% after preservation for 5 days (Day 5), in the case of using the first generation preservation solution. On the other hand, the PAC-1/P-selectin positive ratio in the case of using the preservation solution supplemented with the water-soluble vitamin group was 36.8% even after preservation for 5 days. Furthermore, the PAC-1/P-selectin positive ratio in the case of using the preservation solution supplemented with the water-soluble vitamin group (except for VB2) was elevated to 43.0% after preservation for 5 days. These results showed the possibility that the effect of maintaining platelet functions by VC and VB3 is inhibited by VB2.

Example 15

[Long-term preservation of human mesenchymal stem cells]

(15-1) Preparation of mesenchymal stem cell preservation solution

A CSP-01 solution was prepared according to the description of (10-1) of Example 10. A VC formulation (1000 mg/L, manufactured by Sawai Pharmaceutical Co., Ltd.) and nicotinic acid (400 mg/L, manufactured by TOA EIYO Ltd.) were added to the CSP-01 solution to prepare a mesenchymal stem cell preservation solution (in accordance with the claims). A vehicle distilled water (Otsuka Distilled Water; manufactured by Otsuka Pharmaceutical Factory) was added instead of VC and nicotinic acid to the CSP-01 solution to prepare a control preservation solution.

(15-2) Preservation of mesenchymal stem cells

Human bone marrow-derived mesenchymal stem cells (manufactured by Lonza Group AG) were suspended (5 $\times$ 10$^5$ cells/mL) using each preservation solution prepared in the aforementioned (15-1). The liquid suspension was left standing at 5°C for 1, 2, 4, 7, 14, 21, 28, 35 and 63 days. Then, the total cell number and a dead cell number were counted under a microscope. Cell viability (%) and a viable cell recovery ratio (%) at each point in time were calculated according to the equations 1 and 2 described in (10-2) of Example 10.

The results are shown in Figure 23. It was revealed that the cell viability and the viable cell recovery ratio in the case of using the control preservation solution (in the diagram "+Vehicle") were rapidly decreased after preservation for 2 days. On the other hand, no drastic decrease in cell viability and viable cell recovery ratio was found in the case of using the preservation solution supplemented with VC and nicotinic acid (in the diagram, "+VC+nicotinic acid"), demonstrating that the same level as that before preservation was maintained even after 35 days. Significantly high cell viability and viable cell recovery ratio were exhibited even after preservation for 63 days in the case of using the preservation solution supplemented with VC and nicotinic acid as compared with the control preservation solution. These results showed that VC and nicotinic acid also exert an excellent effect on the preservation of mesenchymal stem cells over a long period of time.

Example 16

[Long-term preservation of human mesenchymal stem cells]

(16-1) Preparation of mesenchymal stem cell preservation solution

A VC formulation (1000 mg/L, manufactured by Sawai Pharmaceutical Co., Ltd.) and nicotinic acid (400 mg/L, manufactured by TOA EIYO Ltd.) were added to the CSP-01 solution or a lactated Ringer's solution (Lactec infusion solution; manufactured by Otsuka Pharmaceutical Factory) to prepare the following four types of mesenchymal stem cell preservation solutions.

CSP-01
CSP-01 + VC + nicotinic acid (in accordance with the claims)
Lactated Ringer's solution
Lactated Ringer's solution + VC + nicotinic acid (in accordance with the claims)

(16-2) Preservation of mesenchymal stem cells

Human adipose-derived mesenchymal stem cells (manufactured by Lonza Group AG) were suspended ($5 \times 10^5$ cells/mL) using each preservation solution prepared in the aforementioned (16-1). The liquid suspension was left standing at 5°C for 7, 14, 21 and 28 days. Then, the total cell number and a dead cell number were counted under a microscope. Cell viability (%) and a viable cell recovery ratio (%) at each point in time were calculated according to the equations 1 and 2 described in (10-2) of Example 10.
The results are shown in Figure 24. The cell viability and the viable cell recovery ratio in the case of using CSP-01 or the lactated Ringer's solution alone was markedly decreased after preservation for 7 days. On the other hand, it was revealed that decrease in cell viability and viable cell recovery ratio was suppressed in the case of using the preservation solution supplemented with VC and nicotinic acid. It was revealed that both the cell viability and the viable cell recovery ratio were kept high even after preservation for 28 days, particularly, in the case of using CSP-01 + VC + nicotinic acid. It was also revealed that both the cell viability and the viable cell recovery ratio were kept high even after preservation for 14 days in the case of using lactated Ringer's solution + VC + nicotinic acid. These results showed that VC and nicotinic acid also exert an excellent effect on the long-term preservation of human adipose-derived mesenchymal stem cells.

Example 17

[Long term preservation of human mesenchymal stem cells]

(17-1) Preparation of mesenchymal stem cell preservation solution

A VC formulation (1000 mg/L, manufactured by Sawai Pharmaceutical Co., Ltd.) and/or nicotinic acid (400 mg/L, manufactured by TOA EIYO Ltd.) were added to the CSP-01 solution, and a sodium bicarbonate formulation (MEYLON Injection 8.4%, manufactured by Otsuka Pharmaceutical Factory) was further added thereto to adjust pH to 7.0 to 7.3. For a control preservation solution, a sodium bicarbonate formulation (MEYLON Injection 8.4%, manufactured by Otsuka Pharmaceutical Factory) was added to the CSP-01 solution to adjust pH to 7.0 to 7.3. In this way, the following four mesenchymal stem cell preservation solutions were prepared.

CSP-01
CSP-01 + VC
CSP-01 + nicotinic acid
CSP-01 + VC + nicotinic acid (in accordance with the claims)

(17-2) Preservation of mesenchymal stem cells

Human adipose-derived mesenchymal stem cells (manufactured by Lonza Group AG) were suspended ($5 \times 10^5$ cells/mL) using each preservation solution prepared in the aforementioned (17-1). The liquid suspension was left standing at 5°C for 7, 14, 21 and 28 days. Then, the total cell number and a dead cell number were counted under a microscope. Cell viability (%) and a viable cell recovery ratio (%) at each point in time were calculated according to the equations 1 and 2 described in (10-2) of Example 10.
The results are shown in Figure 25. The cell viability and the viable cell recovery ratio were linearly decreased in the case of using CSP-01 + nicotinic acid, as in the control (CSP-01). On the other hand, decrease in cell viability and viable cell recovery ratio was significantly suppressed in the case of using CSP-01 + VC. The cell viability and the viable cell recovery ratio were more markedly improved in the case of using CSP-01 + VC + nicotinic acid. These results showed that VC alone is effective for the long-term preservation of mesenchymal stem cells, while use of VC and nicotinic acid in combination exerts a much better effect.

Example 18

[Preservation of neonatal porcine bone marrow mesenchymal stem cells]

(18-1) Preparation of mesenchymal stem cell preservation solution

A VC formulation (1000 mg/L, manufactured by Sawai Pharmaceutical Co., Ltd.) and nicotinic acid (400 mg/L, manufactured by TOA EIYO Ltd.) were added to the CSP-01 solution to prepare a mesenchymal stem cell preservation solution (CSP-01 + VC + nicotinic acid) (in accordance with the claims). The CSP-01 solution was used alone as a control preservation solution.

(18-2) Preservation of mesenchymal stem cells

Neonatal porcine bone marrow-derived mesenchymal stem cells (np mesenchymal stem cells) were prepared according to the method of Nishimura et al. (Xenotransplantation. 2019 May; 26 (3): e12501). The np mesenchymal stem cells were suspended ($5 \times 10^5$ cells/mL) using each preservation solution prepared in the aforementioned (18-1). The liquid suspension was left standing at 5°C for 7, 14, 21 and 28 days. Then, the total cell number and a dead cell number were counted under a microscope. Cell viability (%) and a viable cell recovery ratio (%) at each point in time were calculated according to the equations 1 and 2 described in (10-2) of Example 10.
The results are shown in Figure 26. The viability and viable cell recovery ratio of the np mesenchymal stem cells in any of the preservation periods were improved in the case of using CSP-01 + VC + nicotinic acid as compared with the control (CSP-01). It was revealed that CSP-01 + VC + nicotinic acid kept the viability and viable cell recovery ratio of the np mesenchymal stem cells high even after preservation for 28 days. These results showed that a preservation solution supplemented with VC and nicotinic acid also exerts an excellent effect on the preservation of np mesenchymal stem cells.

Example 19

[Preservation of T cells]

(19-1) Preparation of T cell preservation solution

A VC formulation (1000 mg/L, manufactured by Sawai Pharmaceutical Co., Ltd.), nicotinic acid (400 mg/L, manufactured by TOA EIYO Ltd.) and/or glucose (80 mg/dL, manufactured by Otsuka Pharmaceutical Factory) was added to a lactated Ringer's solution (Lactec infusion solution; manufactured by Otsuka Pharmaceutical Factory) to prepare the following eight types of T cell preservation solutions.

LR
LR + glucose
LR + VC
LR + nicotinic acid
LR + VC + nicotinic acid(in accordance with the claims)
LR + VC + glucose
LR + nicotinic acid + glucose
LR + VC + nicotinic acid + glucose(in accordance with the claims)

(19-2) Preservation of T cells

Commercially available frozen CD8-positive T cells (manufactured by VERITAS Corp.) were thawed, washed with a lymphocyte culture medium (LGM3, manufactured by Lonza Group AG), and then incubated for 1 hour (37°C, 5% $CO_2$). A necessary amount of the CD8-positive T cells was separated and centrifuged for 10 minutes ($300 \times g$, room temperature). After removal of the supernatant, the cells were floated using TLY CULTURE Kit 25 (manufactured by GC Lymphotec Inc.) and cultured under conditions of 37°C and 5% $CO_2$ for proliferation (T cell concentration: approximately $8 \times 10^5$ cells/5 ml). 7 days after the start of expansion culture, the cells were washed with PBS(-), dispensed to STEMFULL tubes (manufactured by Sumitomo Bakelite Co., Ltd.), and centrifuged for 10 minutes ($300 \times g$, room temperature). After removal of the supernatant, the cells were suspended by the addition of each preservation solution prepared in the aforementioned (19-1) (T cell concentration: approximately $5 \times 10^5$ cells/1 ml). At the point in time when the aforementioned liquid suspension of the cells was preserved at 5°C for 24 hours, and the point in time when the liquid suspension was then further preserved at 25°C for 6 hours (after preservation for a total of 30 hours), the total cell number and a dead cell number were counted under a microscope. Cell viability (%) and a viable cell recovery ratio (%) at each point in time were calculated according to the equations 1 and 2 described in (10-2) of Example 10.

The T cell viability after preservation is shown in Figure 27. As shown in the upper graph of Figure 27, it was revealed that the viability after preservation at 5°C for 24 hours was significantly elevated in the case of using the VC-supplemented preservation solution (LR + VC, LR + VC + nicotinic acid, LR + VC + glucose, and LR + VC + nicotinic acid + glucose) as compared with the LR preservation solution. As shown in the lower graph of Figure 27, the viability after preservation at 5°C for 24 hours + at 25°C for 6 hours was significantly elevated in the case of using the preservation solution supplemented with VC and glucose (LR + VC + glucose and LR + VC + nicotinic acid + glucose) as compared with the LR preservation solution, and tended to be elevated in the case of using the preservation solution supplemented with VC and nicotinic acid (LR + VC + nicotinic acid).

The viable cell recovery ratio of T cells after preservation is shown in Figure 28. As shown in the upper graph of Figure 28, it was revealed that the viable cell recovery ratio after preservation at 5°C for 24 hours was significantly elevated in the case of using the VC-supplemented preservation solution (LR + VC, LR + VC + nicotinic acid, LR + VC + glucose, and LR + VC + nicotinic acid + glucose) as compared with the LR preservation solution. Among them, the preservation solution supplemented with VC and glucose in combination (LR + VC + glucose and LR + VC + nicotinic acid + glucose) exhibited a more marked effect of improving a viable cell recovery ratio. As shown in the lower graph of Figure 28, the viable cell recovery ratio after preservation at 5°C for 24 hours + at 25°C for 6 hours was significantly elevated in the case of using the preservation solution supplemented with VC and glucose (LR + VC + glucose and LR + VC + nicotinic acid + glucose) as compared with the LR preservation solution, and tended to be elevated in the case of using the preservation solution supplemented with VC and nicotinic acid (LR + VC + nicotinic acid).

These results showed that use of VC and nicotinic acid in combination improves both viability and a viable cell recovery ratio, as compared with use of each alone, under conditions for actual T cell transplantation in which T cells are preserved at 5°C and further preserved with the temperature changed to 25°C. It was also revealed that use of VC and glucose in combination further improves viability and a viable cell recovery ratio.

**Industrial Applicability**

The present invention enables platelets to be preserved under shaking for at least 10 days while maintaining their functions, and as such, is useful for preparing a platelet formulation for disease or wound treatment. The present invention also enables mesenchymal stem cells, megakaryocytes, T cells, etc. to be preserved over a long period of time by non-frozen preservation, and as such, is useful in the field of transplantation medicine for regenerative medicine or the like and in the field of cancer treatment.

**Claims**

1. Use of a preservation solution for preserving a platelet or a megakaryocyte for 1 to 15 days at 0 to 40°C, wherein the preservation solution comprises 10 to 5000 mg/L niacin or a salt of niacin, and 10 to 8000 mg/L of ascorbic acid or a salt of ascorbic acid, and does not comprise vitamin B2 or a salt of vitamin B2.

2. The use according to claim 1, wherein a concentration of niacin or the salt of niacin is 30 to 3000 mg/L.

3. The use according to Claim 1, wherein a concentration of niacin or the salt of niacin is 120 to 1200 mg/L.

4. The use according to any one of claims 1 to 3, wherein a concentration of ascorbic acid or the salt of ascorbic acid is 30 to 6000 mg/L.

5. The use according to any one of claims 1 to 3, wherein a concentration of ascorbic acid or the salt of ascorbic acid is 300 to 3000 mg/L.

6. The use according to any one of claims 1 to 5, wherein the platelet is a purified platelet obtained by a method comprising the following (A) and (B):

   (A) a condensation step of condensing a culture product of megakaryocytes; and
   (B) a centrifugation step of centrifuging a platelet from the obtained condensed product.

7. The use according to any one of claims 1 to 6, wherein the preservation solution further comprises albumin.

8. The use according to claim 7, wherein a concentration of albumin is 1.25 to 10% (w/v).

9. The use according to any one of claims 1 to 8, wherein the preservation solution further comprises sugar.

10. The use according to claim 9, wherein the sugar is glucose.

11. The use according to any one of claims 1 to 10, for preserving a platelet or a megakaryocyte for 5 to 10 days.

12. Use of a preservation solution for preserving a stem cell or an immunocyte for 1 to 63 days at 0 to 40°C, wherein the preservation solution comprises 10 to 5000 mg/L of niacin or a salt of niacin, 10 to 8000 mg/L of ascorbic acid or a salt of ascorbic acid, and trehalose in an isotonic solution.

13. The use according to claim 12, wherein the stem cell is a mesenchymal stem cell.

14. The use according to claim 12, wherein the immunocyte is a T cell.

15. The use according to any one of claims 12 to 14, wherein the isotonic solution is a lactated Ringer's solution.

16. The use according to any one of claims 12 to 15, wherein the isotonic solution further comprises dextran.

**Patentansprüche**

1. Verwendung einer Konservierungslösung zur Konservierung eines Thrombozyten oder eines Megakaryozyten für 1 bis 15 Tage bei 0 bis 40°C, wobei die Konservierungslösung 10 bis 5000 mg/l Niacin oder ein Salz von Niacin und 10 bis 8000 mg/l Ascorbinsäure oder ein Salz von Ascorbinsäure umfasst und kein Vitamin B2 oder Salz von Vitamin B2 umfasst.

2. Verwendung nach Anspruch 1, wobei eine Konzentration von Niacin oder des Salzes von Niacin 30 bis 3000 mg/l beträgt.

3. Verwendung nach Anspruch 1, wobei eine Konzentration von Niacin oder des Salzes von Niacin 120 bis 1200 mg/l beträgt.

4. Verwendung nach einem der Ansprüche 1 bis 3, wobei eine Konzentration von Ascorbinsäure oder des Salzes von Ascorbinsäure 30 bis 6000 mg/l beträgt.

5. Verwendung nach einem der Ansprüche 1 bis 3, wobei eine Konzentration von Ascorbinsäure oder des Salzes von Ascorbinsäure 300 bis 3000 mg/l beträgt.

6. Verwendung nach einem der Ansprüche 1 bis 5, wobei der Thrombozyt ein gereinigter Thrombozyt ist, der durch ein Verfahren erhalten wird, das die folgenden (A) und (B) umfasst:

   (A) einen Kondensationsschritt des Kondensierens eines Kulturprodukts von Megakaryozyten; und
   (B) einen Zentrifugationsschritt des Zentrifugierens eines Thrombozyten von dem erhaltenen kondensierten Produkt.

7. Verwendung nach einem der Ansprüche 1 bis 6, wobei die Konservierungslösung ferner Albumin umfasst.

8. Verwendung nach Anspruch 7, wobei eine Konzentration von Albumin 1,25 bis 10% (Gew./Vol.) beträgt.

9. Verwendung nach einem der Ansprüche 1 bis 8, wobei die Konservierungslösung ferner Zucker umfasst.

10. Verwendung nach Anspruch 9, wobei der Zucker Glukose ist.

11. Verwendung nach einem der Ansprüche 1 bis 10 zur Konservierung eines Thrombozyten oder eines Megakaryozyten für 5 bis 10 Tage.

12. Verwendung einer Konservierungslösung zur Konservierung einer Stammzelle oder einer Immunzelle für 1 bis 63 Tage bei 0 bis 40°C, wobei die Konservierungslösung 10 bis 5000 mg/l Niacin oder ein Salz von Niacin, 10 bis 8000 mg/l Ascorbinsäure oder ein Salz von Ascorbinsäure und Trehalose in einer isotonischen Lösung umfasst.

13. Verwendung nach Anspruch 12, wobei die Stammzelle eine mesenchymale Stammzelle ist.

14. Verwendung nach Anspruch 12, wobei die Immunzelle eine T-Zelle ist.

15. Verwendung nach einem der Ansprüche 12 bis 14, wobei die isotonische Lösung eine Ringerlaktatlösung ist.

16. Verwendung nach einem der Ansprüche 12 bis 15, wobei die isotonische Lösung ferner Dextran umfasst.

**Revendications**

1. Utilisation d'une solution de conservation pour conserver une plaquette ou un mégacaryocyte pendant 1 à 15 jours à 0 à 40 °C, la solution de conservation comprenant 10 à 5 000 mg/L de niacine ou d'un sel de niacine, et 10 à 8 000 mg/L d'acide ascorbique ou d'un sel d'acide ascorbique, et ne comprenant ni vitamine B2 ni sel de vitamine B2.

2. Utilisation selon la revendication 1, une concentration de niacine ou du sel de niacine étant de 30 à 3 000 mg/L.

3. Utilisation selon la revendication 1, une concentration de niacine ou du sel de niacine étant de 120 à 1 200 mg/L.

4. Utilisation selon l'une quelconque des revendications 1 à 3, une concentration d'acide ascorbique ou du sel d'acide ascorbique étant de 30 à 6 000 mg/L.

5. Utilisation selon l'une quelconque des revendications 1 à 3, une concentration d'acide ascorbique ou du sel d'acide ascorbique étant de 300 à 3 000 mg/L.

6. Utilisation selon l'une quelconque des revendications 1 à 5, la plaquette étant une plaquette purifiée obtenue par un procédé comprenant les étapes (A) et (B) suivantes :

   (A) une étape de condensation consistant à condenser un produit de culture de mégacaryocytes ; et
   (B) une étape de centrifugation consistant à centrifuger une plaquette à partir du produit condensé obtenu.

7. Utilisation selon l'une quelconque des revendications 1 à 6, la solution de conservation comprenant en outre de l'albumine.

8. Utilisation selon la revendication 7, une concentration d'albumine étant de 1,25 à 10 % (p/v).

9. Utilisation selon l'une quelconque des revendications 1 à 8, la solution de conservation comprenant en outre un sucre.

10. Utilisation selon la revendication 9, le sucre étant le glucose.

11. Utilisation selon l'une quelconque des revendications 1 à 10, pour conserver une plaquette ou un mégacaryocyte pendant 5 à 10 jours.

12. Utilisation d'une solution de conservation pour conserver une cellule souche ou un immunocyte pendant 1 à 63 jours à 0 à 40 °C, la solution de conservation comprenant 10 à 5 000 mg/L de niacine ou d'un sel de niacine, 10 à 8 000 mg/L d'acide ascorbique ou d'un sel d'acide ascorbique, et du tréhalose en solution isotonique.

13. Utilisation selon la revendication 12, la cellule souche étant une cellule souche mésenchymateuse.

14. Utilisation selon la revendication 12, l'immunocyte étant un lymphocyte T.

15. Utilisation selon l'une quelconque des revendications 12 à 14, la solution isotonique étant une solution de Ringer lactatée.

16. Utilisation selon l'une quelconque des revendications 12 à 15, la solution isotonique comprenant en outre du dextrane.

[Fig. 1]

[Fig. 2]

(Mean ± S.D. n=3)

[Fig. 3]

[Fig. 4]

[Fig. 5]

[Fig. 6]

[Fig. 7]

[Fig. 8]

|  | First generation | | First generation + VC | | Second generation (First generation + VC + VB3) | |
|---|---|---|---|---|---|---|
|  | plt/mL | (%) | plt/mL | (%) | plt/mL | (%) |
| Day 1 (n=1) | $1.01 \times 10^9$ | NA | $1.10 \times 10^9$ | NA | $1.03 \times 10^9$ | NA |
| Day 5 (n=1) | $8.64 \times 10^8$ | 85.4 | $8.64 \times 10^8$ | 78.8 | $8.93 \times 10^8$ | 86.5 |
| Day 10 (n=1) | $8.80 \times 10^8$ | 86.8 | $9.90 \times 10^8$ | 90.0 | $9.40 \times 10^8$ | 91.4 |

[Fig. 9]

TRAP-6

Legend:
□ Day1
▨ Conventional preservation solution (Day5)
▦ Novel preservation solution (Day5)
▧ Conventional preservation solution (Day10)
■ Novel preservation solution (Day10)

y-axis: Maximum aggregation ratio (%)
x-axis: µM (10, 20, 40)

EP 3 940 061 B1

[Fig. 10]

Collagen

[Fig. 11]

ADP

[Fig. 12]

## Collagen/ADP

[Fig. 13]

[Fig. 14]

EP 3 940 061 B1

[Fig. 15]

[Fig. 16]

| Group | Animal No. | Bleeding time (sec) |
|---|---|---|
| Vehicle | 101 | 600 |
| | 102 | 600 |
| | 103 | 600 |
| | 104 | 600 |
| | 105 | 600 |
| | Mean | 600 |
| | S.E. | 0 |
| Platelets | 201 | 130 |
| | 202 | 474 |
| | 203 | 397 |
| | 204 | 362 |
| | 205 | 592 |
| | Mean | 391 |
| | S.E. | 76 |

[Fig. 17]

### Cell viability after preservation at 5°C

— +VC+nicotinic acid
● +Vehicle

### Viable cell recovery ratio after preservation at 5°C

Data represent mean ± SD (n=5).

[Fig. 18]

[Fig. 19]

Viability (%) 5°C 0hr

Viability (%) 5°C 48hr

Student's t test
n.s.: No significant difference
**:p<0.01
***:p<0.001

[Fig. 20]

[Fig. 21]

[Fig. 22]

ADP/TRAP-6 stimulation

ATR stimulation
Double positive %

First generation — 35.6 %

First generation + water-soluble vitamin — 36.8 %

First generation + water-soluble vitamin (except for VB2) — 43.0 %

No stimulation

No stimulation
P-Selectin positive %

First generation — 27.4 %

First generation + water-soluble vitamin — 34.9 %

First generation + water-soluble vitamin (except for VB2) — 24.0 %

First generation + water-soluble vitamin — 42.9 %

Day 1 (n=1)

Day 5 (n=2 Mean)

[Fig. 23]

Mean ± standard deviation (n=5).
*;p<0.05, **;p<0.01, ***;p<0.001, Student's t test

[Fig. 24]

Mean ± standard deviation (n=6)
\*\*\*;p<0.001, Student's t test

Mean ± standard deviation (n=6)
\*;p<0.05, \*\*\*;p<0.001, Student's t test

[Fig. 25]

Mean ± standard deviation (n=4)

*;p<0.05, **;p<0.01, ***;p<0.001 vs CSP-01, Dunnett's multiple comparison test

[Fig. 26]

Mean ± standard deviation (n=6)
***;p<0.001, Student's t test

[Fig. 27]

5°C24hr

Mean±SD(n=3)
**;p<0.01, ***;p<0.001 vs LR Dunnett's multiple comparison test

5°C24hr+25°C6hr

Mean±SD(n=3)
**;p<0.05, ***;p<0.01 vs LR Dunnett's multiple comparison test

[Fig. 28]

5°C24hr

Mean±SD(n=3)
**;p<0.01, ***;p<0.001 vs LR Dunnett's multiple comparison test

5°C24hr+25°C6hr

Mean±SD(n=3)
**;p<0.01, vs LR Dunnett's multiple comparison test

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017077964 A
- JP 2018034667 W
- WO 2017073656 A
- JP 2018153196 A
- JP 2012115253 A
- WO 2014208053 A

**Non-patent literature cited in the description**

- **TAKAYAMA**. *Blood*, 2008, vol. 111, 5298-5306
- **TYLER**. *Blood*, 2013, vol. 121, 308-317
- **CAMPBELL**. *Clin vaccine immunol*, 2009, vol. 16, 1648-53
- **TAKAYAMA et al.** *J. Exp. Med.*, 2010, vol. 13, 2817-2830
- **KOBAYASHI et al.** *Cell*, 2010, vol. 142 (5), 787-799
- *Japanese Red Cross Society, the package insert of Irradiated Washed Platelets-LR "NISSEKI*, March 2016
- *Japanese Journal of Transfusion and Cell Therapy*, 2013, vol. 59 (3), 492-498